(19) Europäisches Patentamt
European Patent Office
Office européen des brevets

(11) EP 2 086 525 B1

(12) **EUROPEAN PATENT SPECIFICATION**

(45) Date of publication and mention of the grant of the patent:
**06.10.2010 Bulletin 2010/40**

(21) Application number: **07862155.4**

(22) Date of filing: **20.11.2007**

(51) Int Cl.:
***A61K 31/00*** $^{(2006.01)}$ ***A61P 35/00*** $^{(2006.01)}$

(86) International application number:
**PCT/US2007/024271**

(87) International publication number:
**WO 2008/063644 (29.05.2008 Gazette 2008/22)**

(54) **METHOD OF RADIO-SENSITIZING TUMORS USING A RADIO-SENSITIZING AGENT**

VERFAHREN ZUR STRAHLENSENSIBILISIERUNG VON TUMOREN MIT EINEM STRAHLENSENSIBILISIERUNGSMITTEL

PROCÉDÉ DE RADIOSENSIBILISATION DES TUMEURS À L'AIDE D'UN AGENT RADIOSENSIBILISATEUR

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **20.11.2006 US 860036 P**

(43) Date of publication of application:
**12.08.2009 Bulletin 2009/33**

(73) Proprietor: **Cephalon, Inc.**
**Frazer, PA 19355 (US)**

(72) Inventors:
- **DIEBOLD, James L.**
  **Eagleville, PA 19403 (US)**
- **HUDKINS, Robert L.**
  **Chester Springs, PA 19425-2331 (US)**
- **MIKNYOCZKI, Sheila J.**
  **Easton, PA 18040 (US)**
- **RUGGERI, Bruce**
  **West Chester, PA 19380 (US)**

(74) Representative: **Hallybone, Huw George**
**Carpmaels & Ransford**
**One Southampton Row**
**London**
**WC1B 5HA (GB)**

(56) References cited:
**WO-A-01/85686 US-A1- 2006 276 497**

- **GRIFFIN R J ET AL: "THE ROLE OF INHIBITORS OF POLY(ADP-RIBOSE) POLYMERASE AS RESISTANCE-MODIFYING AGENTS IN CANCER THERAPY" BIOCHIMIE, MASSON, PARIS, FR, vol. 77, no. 6, 1995, pages 408-422, XP001086401 ISSN: 0300-9084 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 086 525 B1

## Description

### Field of the Invention

The present invention relates to a method of treating cancer using PARP inhibitors as radio-sensitization agents of tumors. Specifically the present invention relates to a method of radio-sensitization of tumors using a compound of Formula (I)

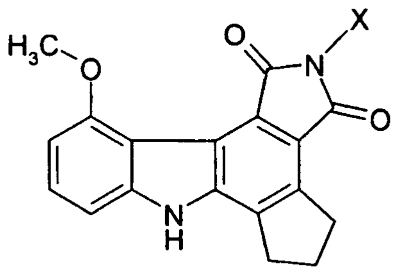

(I)

or a pharmaceutically acceptable salt form thereof. The present invention also relates to a pharmaceutical compositions of PARP inhibitors for radiosensitizing tumors.

### Background of the Invention

Radiation is a cytotoxic treatment modality that induces cellular damage by creating DNA strand breaks. Poly (ADP-ribose) polymerase 1 (PARP-1) a nuclear zinc finger DNA binding protein which is activated by and implicated in DNA radiation induced-damage and repair. PARP binds to DNA strand breaks which may serve to protect them from nuclease attack or recombination. Since PARP acts to aid in DNA repair, inhibitors have the potential to enhance the chemo- and radio-sensitization of cytotoxic agents (Curtin, 2005).

The most significant cause for treatment failure and cancer mortality is radio/chemo-resistance. Agents to overcome cancer cell resistance to cytotoxic agents may be a key factor in successful cancer therapy. The potential application of PARP inhibitors therapeutically as chemo- and radio-sensitizers has, until relatively recently, been limited by the potency, selectivity, and pharmaceutic properties of these agents (Griffin et al., 1998; Bowman, et al., 1998; Bowman et al., 2001, Chen & Pan, 1998; Delany et al., 2000; Griffin et al., 1995; Lui, et al., 1999). Recently, more potent and selective PARP inhibitors (benzimidazole-4-carboxamides and quinazolin-4-[3*H*]-ones) have been developed that have demonstrated the ability to potentiate the effects of radiation and of chemotherapeutic agents such as camptothecin (CPT), topotecan, irinotecan, cisplatin, etoposide, bleomycin, BCNU, and temozolomide (TMZ) *in vitro* and *in vivo* using both human and murine tumor models of leukemia, lymophma metastases to the central nervous system, colon, lung and breast carcinomas agents (Griffin et al., 1998; Bowman, et al., 1998; Bowman et al., 2001, Chen & Pan, 1998; Delany et al., 2000; Griffin et al., 195; Lui, et al., 1999, Tentori, et al., 2002). A PARP inhibitor that is able to sensitize tumor cells to the actions of different classes of chemotherapeutic agents and/or radiation could increase the success rate of established cancer therapies.

PARP-1 is a 116kD nuclear zinc finger DNA binding protein that uses NAD+ as a substrate to transfer ADP-ribose onto acceptor proteins such as histones polymerases, ligases, and PARP itself (automodification) (Griffin et al., 1998; Tentori, et al., 2002; Baldwin et al., 2002). PARP-1 belongs to a family of proteins that currently includes 18 members, of these PARP-1 and PARP-2 are the only enzymes activated by DNA damage (Curtin, 2005; Tentori, et al., 2002). Activation of PARP-2 may also induce pro-inflammatory activity (Jagtap and Szabo, 2005), indicating that inhibition of PARP-2 in tumor cells may be of additional therapeutic benefit. Although the pathophysiological and physiological process modulated by the various PARP isoforms are the subject of extensive study (Ame et al., 2004), the best characterized member of this family, and the major focus of targeted drug discovery efforts therapeutically in oncology, is PARP-1.

PARP is active in the regulation of many different biological processes, including protein expression at the transcriptional level, replication and differentiation, telomerase activity, and cytoskeletal organization. However, it is the role PARP plays in DNA repair and maintenance of genomic integrity that is of interest for the use of PARP inhibitors as chemo/radio-sensitizing agents (Smith, 2001). This role is illustrated via the use of PARP-1 deficient cells which

demonstrate delayed base excision repair and a high frequency of sister chromatid exchange upon exposure to ionizing radiation or treatment with alkylating agents. In addition, high levels of ionizing radiation and alkylating agents elicit higher lethality in PARP-1 deficient mice as compared to wild type mice (Smith, 2001; Virag & Szabo, 2002).

Among the members of the PARP family, PARP-1 (and PARP-2) is specifically activated by, and implicated in, the repair of DNA strand breaks caused directly by ionizing radiation, or indirectly following enzymatic repair of DNA lesions due to methylating agents, topoisomerase I inhibitors, and other chemotherapeutic agents such as cisplatin and bleomycin (Griffin et al., 1998; Delany et al., 2000; Tentori et al., 2002; de Murcia et al., 1997). There is a substantial body of biochemical and genetic evidence demonstrating that PARP-1 plays a role in cell survival and repair following sub-lethal massive DNA damage. Furthermore, as exemplified by PARP-1 knockout mice, PARP-1 function in the absence of DNA damage is not critical for cell survival has made inhibition of PARP-1 a potentially viable therapeutic strategy for use with chemo- and/or radio-therpy (Delany et al., 2000; Burkle et al., 1993).

Early generations of PARP-1 inhibitors such as 3-aminobenzamide, nicotinamide and related derivatives, potentiated both the *in vitro* and *in vivo* cytotoxic activities of radiation, bleomycin, CPT, cisplatin and TMZ in human and murine tumor models *in vitro* and *in vivo.* The inherent limitations in the potency, selectivity, and deliverability of these compounds precluded assigning unequivocally the potentiation of anti-tumor efficacy observed *in vitro* and *in vivo* to the inhibition of PARP-1 specifically versus non-specific activities of these molecules (Griffin et al., 1998; Griffin et al., 1995; Masuntani et al., 2000; Kato et al., 1988). These issues were influential in the development of more potent and selective structural classes of PARP-1 inhibitors including various benzimidazole-4-carboxamides and quinazolin-4-[3H]-one derivatices. *In vitro* and *In vivo* analyses revealed that these compounds were able to potentiate the efficacy of chemotherapeutic agents using both human and murine tumor models (Griffin et al., 1998; Bowman, et al., 1998; Bowman et al., 2001; Chen & Pan, 1998; Delany et al., 2000; Griffin et al., 1995; Liu, et al., 1999).

PCT publication WO2001085686, published Nov. 15, 2001, discloses carbazole compounds with PARP inhibitory activity.

There is a need to discover and develop PARP inhibitors as radio-sensitization agents for the treatment of cancer which have high selectivity for PARP, high potency, improved deliverability, and improved tolerability profiles.

**Summary of the Invention**

The present invention provides a method of using a 4-methoxy-carbazole to cause radio-sensitization in tumors by the *in vivo* inhibition of PARP-1. The method comprises a 4-methoxy-carbazole of Formula (Ia):

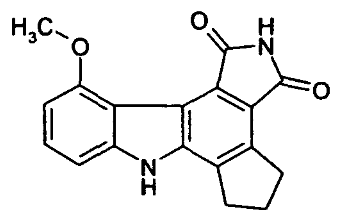

(Ia)

and prodrugs thereof, preferably a Mannich base prodrug thereof, to provide solubility and stability, and to aid in the *in vivo* delivery of the active drug, 7-methoxy-1,2,3,11-tetrahydro-5,11-diaza-benzo[a]trindene-4,6-dione.

The present invention further provides for a method of treating cancer by administering a radiosensitizing agent of Formula (I):

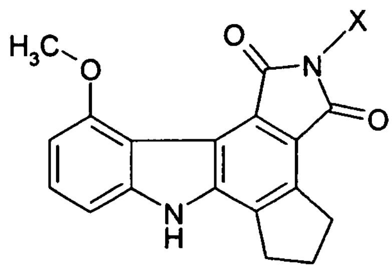

(I)

or a pharmaceutically acceptable salt form thereof, wherein, X is H or a prodrug moiety, as defined herein; to a mammal suffering from cancer and applying ionizing radiation to said mammal tissue.

Another object of the present invention is to provide pharmaceutical compositions comprising the compounds of the present invention wherein the compositions comprise one or more pharmaceutically acceptable excipients and a therapeutically effective amount of at least one of the compounds of the present invention, or a pharmaceutically acceptable salt or ester form thereof.

Another object of the present invention is to provide a compound of Formula (II):

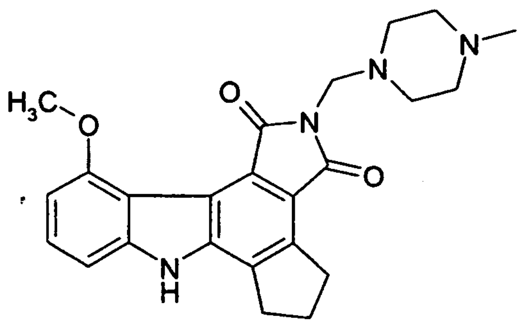

(II)

or a pharmaceutically acceptable salt form thereof.

In another embodiment, the present invention provides use of a compound of Formula (I) for the manufacture of a medicament for the treatment of cancer.

These and other objects, features and advantages of the invention will be disclosed in the following detailed description of the patent disclosure.

**Brief Description of the Diagrams**

Fig.1: Shows the effect of the Mannich base prodrug in combination with Radiation using radio-resistant U87MG glioblastoma xenografts on the growth delay of the tumors.

Fig.2: Magnitude of effect with combination therapy stronger than that achieved with a comparable regimen of radiotherapy or the prodrug only.

Fig.3: Radio-sensitizing Effect of Example 7 in U87MG Human Glioblastoma Xenografts in Nude Mice (Non-optimized Schedule).

Fig. 4 shows a synthetic schematic including a compound within the scope of the present invention and precursors thereto.

Fig.5: Radio-sensitizing Effect of Example 7 administered orally in U87MG Human Glioblastoma Xenografts in Nude Mice.

## Detailed Description of the Invention

In a first embodiment, the present invention provides a method of treating cancer by administering a radiosensitizing agent of Formula (I):

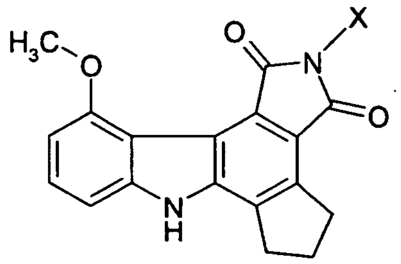

(I)

or a pharmaceutically acceptable salt form thereof, wherein, X is H or a prodrug moiety; to a mammal suffering from cancer and applying ionizing radiation to said mammal tissue.

In a preferred embodiment the radiosensitizing agent is present within or proximate to said tissue increases the efficiency of conversion of said applied ionizing radiation into localized therapeutic effects.

In a preferred embodiment the radiosensitizing agent is present in an amount effective to radiosensitize cancer cells.

In a preferred embodiment the ionizing radiation of said tissue is performed with a dose of radiation effective to destroy said cells.

In a preferred embodiment the ionizing radiation is of clinically acceptable or recommended radiotheraputic protocols for a given cancer type.

In a preferred embodiment the cancer is malignant.

In a preferred embodiment the cancer is benign.

In a preferred embodiment the the prodrug moiety is selected from the group consisting of $-CH_2NR^1R^2$, $-CH_2OC(=O)R^3$, $-CH_2OP(=O)(OH)_2$ and $-C(=O)R^4$; wherein;

- $R^1$ is H or $C_{1-4}$ alkyl;
- $R^2$ is H or $C_{1-4}$ alkyl; alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$ alkyl;
- $R^3$ is selected from the group consisting of $-C_{1-4}$ alkyl-$NR^1R^2$, $-C_{1-4}$ alkyl-$OR^5$, pyridinyl, -phenyl($CH_2NR^1R^2$), and $-CH(R^6)NH_2$;
- $R^4$ is selected from the group consisting of-O-($C_{1-4}$ alkyl)-$NR^1R^2$, -O-($C_{1-4}$ alkyl)-$OR^5$, and $-CH(R^6)NH_2$;
- $R^5$ is H or $C_{1-4}$ alkyl; and
- $R^6$ is the side chain of a naturally occurring amino acid.

In a preferred embodiment the prodrug moiety is $-CH_2NR^1R^2$, $R^1$ is H or $C_{1-4}$ alkyl; $R^2$ is H or $C_{1-4}$ alkyl; and alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$ alkyl.

In a preferred embodiment the prodrug moiety is a Mannich base.

In a preferred embodiment the Mannich base is selected form 4-methyl-piperazin-1-ylmethyl-, morpholin-4-ylmethyl-, and 5-diethylaminomethyl-.

In a preferred embodiment the Mannich base is 4-methyl-piperazin-1-ylmethyl.

In a preferred embodiment the route of administration is intravenous, subcutaneous, oral or intraperitoneally.

In a preferred embodiment the route of administration is intravenous.

In a preferred embodiment the cancer is selected from head and neck squamous cell carcinoma (eye, lip, oral , pharynx, larynx, nasal, carcinoma of the tongue, and esophogeal carcinoma), melanoma, squamous cell carcinoma (epidermis), glioblastoma, astrocytoma, oligodendroglioma, oligoastrocytoma, meningioma, neuroblastoma, rhabdomyosarcoma, soft-tissue sarcomas, osteosarcoma, hematologic malignancy at the cns site, breast carcinoma (ductal and carcinoma in situ), thyroid carcinoma (papillary and follicular), lung carcinoma (bronchioloalveolar carcinoma, small cell

lung carcinoma, mixed small cell/large cell carcinoma, combined small cell carcinoma, non-small cell lung carcinoma, squamous cell carcinoma, large cell carcinoma, and adenocarcinoma of the lung), hepatocellular carcinoma, colo-rectal carcinoma, cervical carcinoma, ovarian carcinoma, prostatic carcinoma, testicular carcinoma, gastric carcinoma, pancreatic carcinoma, cholangiosarcoma, lymphoma (Hodgkins and non-Hodgkins types of T-and B-cell origin), leukemia (acute and chronic leukemias of myeloid and lymphoid origins), and bladder carcinoma.

In a preferred embodiment the cancer is selected from head and neck squamous cell carcinoma (eye, lip, oral, pharynx, larynx, nasal, carcinoma of the tongue, and esophogeal carcinoma), melanoma, squamous cell carcinoma (epidermis), glioblastoma, neuroblastoma, rhabdomyosarcoma, lung carcinoma, (bronchioloalveolar carcinoma, small cell lung carcinoma, mixed small cell/large cell carcinoma, combined small cell carcinoma, non-small cell lung carcinoma, squamous cell carcinoma, large cell carcinoma, and adenocarcinoma of the lung), lymphoma (Hodgkins and non-Hodgkins types of T-and B-cell origin), and leukemia (acute and chronic leukemias of myeloid and lymphoid origins).

In a preferred embodiment, the present invention provides a method of treating cancer by administering a radiosensitizing agent of formula 7-methoxy-1,2,3,11-tetrahydro-5,11-diaza-benzo[a]trindene-4,6-dione.

In a preferred embodiment, the present invention provides a method of treating cancer by administering a radiosensitizing agent of formula 7-methoxy-1,2,3,11-tetrahydro-5,11-diaza-benzo[a]trindene-4,6-dione.

In a second embodiment, the present invention provides a pharmaceutical composition for radiosensitizing cancer cells comprising a radiosensitizing amount of a compound of Formula (I):

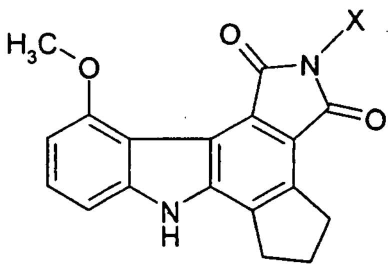

(I)

or a pharmaceutically acceptable salt form thereof, wherein X is H or a prodrug moiety; and a pharmaceutically acceptable carrier.

In a preferred embodiment, the prodrug moiety is selected from the group consisting of $-CH_2NR^1R^2$, $-CH_2OC(=O)R^3$, $-CH_2OP(=O)(OH)_2$ and $-C(=O)R^4$;

- $R^1$ is H or $C_{1-4}$ alkyl;
- $R^2$ is H or $C_{1-4}$ alkyl; alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$alkyl;
- $R^3$ is selected from the group consisting of $-C_{1-4}$ alkyl-$NR^1R^2$, $-C_{1-4}$ alkyl-$OR^5$, pyridinyl, -phenyl($CH_2NR^1R^2$), and $-CH(R^6)NH_2$;
- $R^4$ is selected from the group consisting of -O-($C_{1-4}$ alkyl)-$NR^1R^2$, -O-($C_{1-4}$ alkyl)-$OR^5$, and $-CH(R^6)NH_2$;
- $R^5$ is H or $C_{1-4}$ alkyl; and
- $R^6$ is the side chain of a naturally occurring amino acid. In a preferred embodiment, the prodrug moiety is $-CH_2NR^1R^2$,
- $R^1$ is H or $C_{1-4}$ alkyl;
- $R^2$ is H or $C_{1-4}$ alkyl; and

alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$ alkyl.

In a preferred embodiment, the compound is

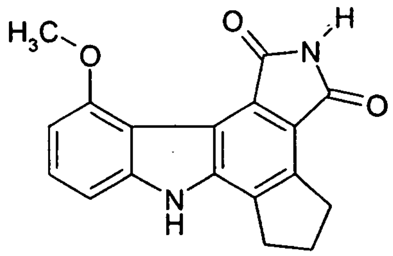

or a pharmaceutically acceptable salt form thereof.

In a preferred embodiment, the compound is

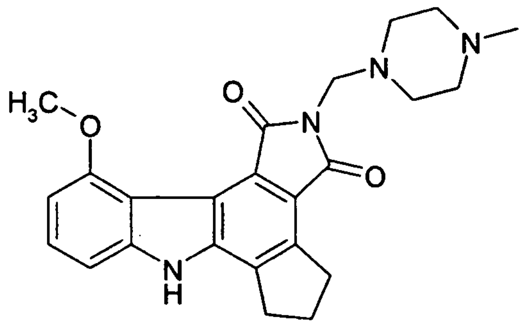

or a pharmaceutically acceptable salt form thereof.

In a third embodiment, the present invention provides for a compound of Formula (II):

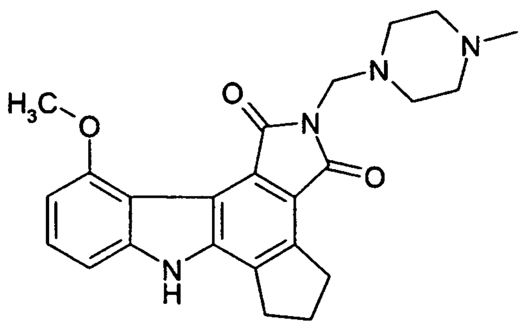

(II)

or a pharmaceutically acceptable salt form thereof.

In a fourth embodiment, the present invention provides use of a compound of Formula (I) for the manufacture of a medicament for the treatment of cancer.

In a preferred embodiment, the present invention provides use of a compound of Formula (II) for the manufacture of a medicament for the treatment of cancer.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable subcombination.

The following terms and expressions contained herein are defined as follows:

As used herein, the term "about" refers to a range of values from ± 10% of a specified value. For example, the phrase "about 50 mg" includes ± 10% of 50, or from 45 to 55 mg.

As used herein, the term "alkyl" refers to a straight-chain, or branched, alkyl group having 1 to 4 carbon atoms, such

as methyl, ethyl, propyl, isopropyl, butyl, isobutyl, sec-butyl, and tert-butyl. A designation such as "$C_1$-$C_4$ alkyl" refers to an alkyl radical containing from 1 to 4 carbon atoms.

As used herein, the term "amino acid" means a molecule containing both an amino group and a carboxyl group. It includes an "α-amino acid" which is well known to one skilled in the art as a carboxylic acid that bears an amino functionality on the carbon adjacent to the carboxyl group. Amino acids can be naturally occurring or non-naturally occurring. "Naturally occurring amino acids" include alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine.

As used herein, the term "heterocyclyl" refers to a 5 or 6 membered cyclic group containing carbon atoms and at least heteroatom selected form O, N, or S, wherein said heterocyclyl group may be saturated or unsauturated and wherein said heterocyclyl group may be substituted or unsubstituted. The nitrogen and sulfur heteroatoms may be optionally oxidized. Examples of heterocyclyl groups include pyrrolyl, pyrrolidinyl, piperidinyl, morpholinyl, thiomorpholinyl, piperazinyl, and methylpiperazinyl.

As used herein, the term "mammal" refers to a warm blooded animal such as a mouse, rat, cat, dog, monkey or human, preferably a human, or a human child, which is afflicted with, or has the potential to be afflicted with, one or more diseases and conditions described herein.

As used herein, a "pharmaceutically acceptable" component is one that is suitable for use with humans and/or animals without undue adverse side effects (such as toxicity, irritation, and allergic response) commensurate with a reasonable benefit/risk ratio.

As used herein, the term "safe and effective amount" refers to the quantity of a component which is sufficient to yield a desired therapeutic response without undue adverse side effects (such as toxicity, irritation, or allergic response) commensurate with a reasonable benefit/risk ratio when used in the manner of this invention. By "therapeutically effective amount" is meant an amount of a compound of the present invention effective to yield the desired therapeutic response. For example, an amount effective to delay the growth of or to cause a cancer, either a sarcoma or lymphoma, or to shrink the cancer or prevent metastasis. The specific safe and effective amount or therapeutically effective amount will vary with such factors as the particular condition being treated, the physical condition of the patient, the type of mammal or animal being treated, the duration of the treatment, the nature of concurrent therapy (if any), and the specific formulations employed and the structure of the compounds or its derivatives.

In the present invention, the term "ionizing radiation" means radiation comprising particles or photons that have sufficient energy or can produce sufficient energy via nuclear interactions to produce ionization (gain or loss of electrons). An exemplary and preferred ionizing radiation is an x-radiation. Means for delivering x-radiation to a target tissue or cell are well known in the art. The amount of ionizing radiation needed in a given cell generally depends on the nature of that cell. Means for determining an effective amount of radiation are well known in the art. Used herein, the term "an effective dose" of ionizing radiation means a dose of ionizing radiation that produces an increase in cell damage or death when given in conjunction with the compounds of the invention.

Dosage ranges for x-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells.

Any suitable means for delivering radiation to a tissue may be employed in the present invention. Common means of delivering radiation to a tissue is by an ionizing radiation source external to the body being treated. Alternative methods for delivering radiation to a tissue include, for example, first delivering *in vivo* a radiolabeled antibody that immunoreacts with an antigen of the tumor, followed by delivering *in vivo* an effective amount of the radiolabeled antibody to the tumor. In addition, radioisotopes may be used to deliver ionizing radiation to a tissue or cell. Additionally, the radiation may be delivered by means of a radiomimetic agent. As used herein a "radiomimetic agent" is a chemotherapeutic agent, for example melphalan, that causes the same type of cellular damage as radiation therapy, but without the application of radiation.

As used herein the term "prodrug moiety" means; the prodrug can be converted under physiological conditions to the biologically active drug by a number of chemical and biological mechanisms. In one embodiment, conversion of the prodrug to the biologically active drug can be accomplished by hydrolysis of the prodrug moiety provided the prodrug moiety is chemically or enzymatically hydrolyzable with water. The reaction with water typically results in removal of the prodrug moiety and liberation of the biologically active drug. Yet another aspect of the invention provides conversion of the prodrug to the biologically active drug by reduction of the prodrug moiety. Typically in this embodiment, the prodrug moiety is reducible under physiological conditions in the presence of a reducing enzymatic process. The reduction preferably results in removal of the prodrug moiety and liberation of the biologically active drug. In another embodiment, conversion of the prodrug to the biologically active drug can also be accomplished by oxidation of the prodrug moiety. Typically in this embodiment, the prodrug moiety is oxidizable under physiological conditions in the presence of an

oxidative enzymatic process. The oxidation preferably results in removal of the prodrug moiety and liberation of the biologically active drug. A further aspect of the invention encompasses conversion of the prodrug to the biologically active drug by elimination of the prodrug moiety. Generally speaking, in this embodiment the prodrug moiety is removed under physiological conditions with a chemical or biological reaction. The elimination results in removal of the prodrug moiety and liberation of the biologically active drug. Of course, any prodrug compound of the present invention may undergo any combination of the above detailed mechanisms to convert the prodrug to the biologically active compound. For example, a particular compound may undergo hydrolysis, oxidation, elimination, and reduction to convert the prodrug to the biologically active compound. Equally, a particular compound may undergo only one of these mechanisms to convert the prodrug to the biologically active compound.

As used herein, "cancer" refers to all types of cancer or neoplasm or malignant or benign tumors found in mammals, including carcinomas and sarcomas. Examples of cancers are cancer of the brain, breast, pancreas, cervix, colon, head & neck, kidney, lung, non-small cell lung, melanoma, mesothelioma, ovary, sarcoma, stomach, uterus and Medulloblastoma.

The term "leukemia" refers broadly to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous), lymphoid (lymphogenous), or monocytic; and (3) the increase or non-increase in the number abnormal cells in the blood-leukemic or aleukemic (subleukemic). The P388 leukemia model is widely accepted as being predictive of in vivo anti-leukemic activity. It is believed that compounds that tests positive in the P388 assay will generally exhibit some level of anti-leukemic activity in vivo regardless of the type of leukemia being treated. Accordingly, the present invention includes a method of treating leukemia, and, preferably, a method of treating acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

The term "sarcoma" generally refers to a tumor which is made up of a substance like the embryonic connective tissue and is generally composed of closely packed cells embedded in a fibrillar or homogeneous substance. Sarcomas which can be treated with 4-methoxy-carbazole and radiotherapy include a chondrosarcoma, cholangiosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, soft-tissue sarcoma, synovial sarcoma, and telangiectaltic sarcoma.

The term "melanoma" is taken to mean a tumor arising from the melanocytic system of the skin and other organs. Melanomas which can be treated with 4-methoxy-carbazole and radiotherapy include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

The term "carcinoma" refers to a malignant new growth made up of epithelial cells tending to infiltrate the surrounding tissues and give rise to metastases. Exemplary carcinomas which can be treated with 4-methoxy-carbazole and radiotherapy include, for example, acinar carcinoma, acinous carcinoma, adenocystic carcinoma, adenoid cystic carcinoma, breast carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bladder carcinoma, bronchioalveolar carcinoma, bronchiolar carcinoma, bronchogenic carcinoma, cerebriform carcinoma, cholangiocellular carcinoma, chorionic carcinoma, colloid carcinoma, colorectual carcinoma, cervical carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gastric carcinoma, gelatiniform carcinoma, gelatinous carcinoma, giant cell carcinoma, carcinoma gigantocellulare, glandular

carcinoma, granulosa cell carcinoma, hair-matrix carcinoma, hematoid carcinoma, hepatocellular carcinoma, Hurthle cell carcinoma, hyaline carcinoma, hypemephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lung carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, ovarian carcinoma, pancreatic carcinoma, prostatic carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, testicular carcincoma, transitional cell carcinoma, thyroid carcinoma, carcinoma tuberosum, tuberous carcinoma, verrucous carcinoma, and carcinoma villosum.

Preferred cancers which can be treated with compounds according to the invention include, head and neck squamous cell carcinoma (eye, lip, oral , pharynx, larynx, nasal, carcinoma of the tongue, and esophogeal carcinoma), melanoma, squamous cell carcinoma (epidermis), glioblastoma, astrocytoma, oligodendroglioma, oligoastrocytoma, meningioma, neuroblastoma, rhabdomyosarcoma, soft-tissue sarcomas, osteosarcoma, hematologic malignancy at the cns site, breast carcinoma (ductal and carcinoma in situ), thyroid carcinoma (papillary and follicular), lung carcinoma (bronchioloalveolar carcinoma, small cell lung carcinoma, mixed small cell/large cell carcinoma, combined small cell carcinoma, non-small cell lung carcinoma, squamous cell carcinoma, large cell carcinoma, and adenocarcinoma of the lung), hepatocellular carcinoma, colo-rectal carcinoma, cervical carcinoma, ovarian carcinoma, prostatic carcinoma, testicular carcinoma, gastric carcinoma, pancreatic carcinoma, cholangiosarcoma, lymphoma (Hodgkins and non-Hodgkins types of T-and B-cell origin), leukemia (acute and chronic leukemias of myeloid and lymphoid origins), and bladder carcinoma.

More preferred cancers which can be treated with compounds according to the invention include, head and neck squamous cell carcinoma (eye, lip, oral, pharynx, larynx, nasal, carcinoma of the tongue, and esophogeal carcinoma), melanoma, squamous cell carcinoma (epidermis), glioblastoma, neuroblastoma, rhabdomyosarcoma, lung carcinoma, (bronchioloalveolar carcinoma, small cell lung carcinoma, mixed small cell/large cell carcinoma, combined small cell carcinoma, non-small cell lung carcinoma, squamous cell carcinoma, large cell carcinoma, and adenocarcinoma of the lung), lymphoma (Hodgkins and non-Hodgkins types of T-and B-cell origin), and leukemia (acute and chronic leukemias of myeloid and lymphoid origins).

As used herein, the term "4-methoxy-carbazole" is used to mean those chemicals having the formula:

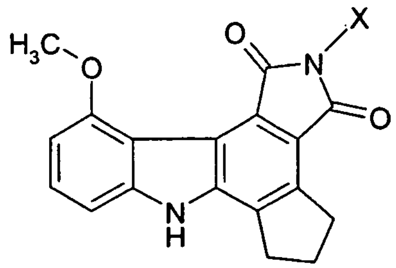

or a pharmaceutically acceptable salt form thereof, wherein, X is H or a prodrug moiety.

The compound of the present invention may contain a prodrug moiety. Examples of a prodrug moiety contemplated by the invention can be selected from phosphate esters, amino acid esters, amino acid amides, aminoalkyl carbamates, alkoxyalkyl carbamates, hydroxyalkyl carbamates, alkoxyalkyl esters, hydroxyalkyl esters, benzoic acid esters, nicotinic esters, piperazine acetates, morpholine acetates, and Mannich bases. Examples of a prodrug moiety contemplated by the invention can be selected from:

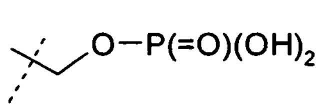 ; 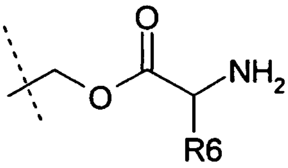 ; 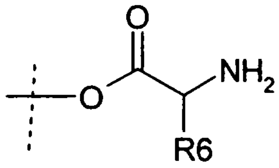 ;

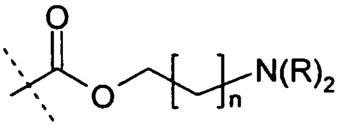 ; 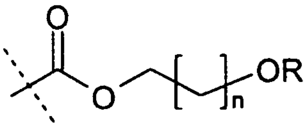 ; 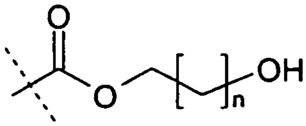 ;

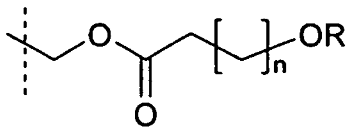 ; 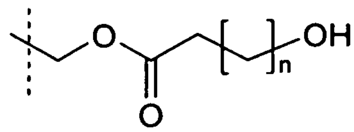 ;

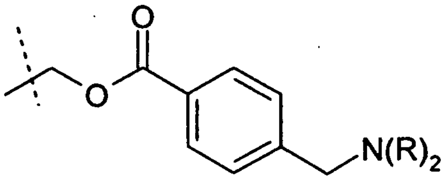 ; 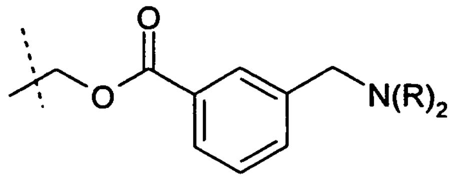 ;

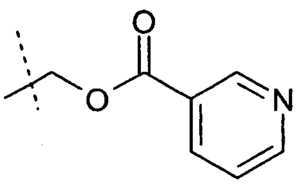 ; 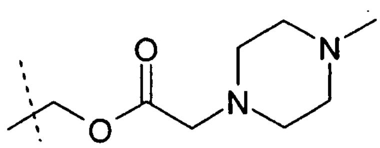 ; 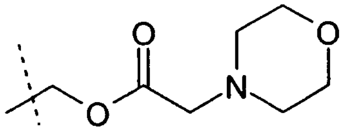 ;

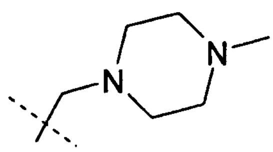 ; 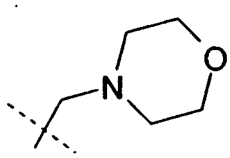 ; and 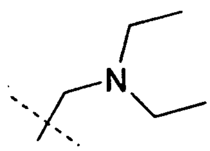 .

A preferred prodrug moiety is a Mannich base. Preferred Mannich bases include, but are not limited to, 4-methyl-piperazin-1-ylmethyl-, morpholin-4-ylmethyl-, and diethylaminomethyl-.

Compounds of the present invention also may take the form of a pharmacologically acceptable salt, hydrate, solvate, or metabolite. Pharmacologically acceptable salts include basic salts of inorganic and organic acids, including but not limited to hydrochloric acid, hydrobromic acid, sulphuric acid, phosphoric acid, methanesulphonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid, ascorbic acid, gluconic acid and the like. When compounds of the invention include an acidic function, such as a carboxy group, then suitable pharmaceutically acceptable cation pairs for the carboxy group are well known to those skilled in the art and include alkaline, alkaline earth, ammonium, quaternary ammonium cations and the like. It is contemplated by the invention that when compounds of the present invention take the form of a pharmacologically acceptable salt, said salt form may be generated *in situ* or as an isolated solid.

The compounds of the present invention, particularly in the form of the salts just described, can be combined with various excipient vehicles and/or adjuvants well known in this art which serve as pharmaceutically acceptable carriers to permit drug administration in the form of, e.g., injections, suspensions, emulsions, tablets, capsules, and ointments. These pharmaceutical compositions, containing a radiosensitizing amount of the described compounds, may be administered by any acceptable means which results in the radiosensitization of hypoxic tumor cells. For warm-blooded animals, and in particular, for humans undergoing radiotherapy treatment, administration can be oral, subcutaneous, intraperitoneally or intravenous. To destroy hypoxic tumor cells, the pharmaceutical composition containing the radiosensitizing agent is administered in an amount effective to radiosensitize the hypoxic tumor cells. The specific dosage administered will be dependent upon such factors as the general health and physical condition of the patient as well as his age and weight, the stage of the patient’s disease condition, and the existence of any concurrent treatments.

The method of administering an effective amount also varies depending on the disorder or disease being treated. It is believed that treatment by intravenous application of the 4-methoxy-carbazole, formulated with an appropriate carrier, additional cancer inhibiting compound or compounds or diluent to facilitate application will be the preferred method of administering the compounds to warm blooded animals.

Compounds described herein may be administered in pure form, combined with other active ingredients, or combined with pharmaceutically acceptable nontoxic excipients or carriers. Oral compositions will generally include an inert diluent carrier or an edible carrier. Pharmaceutically compatible binding agents, and/or adjuvant materials can be included as part of the composition. Tablets, pills, capsules, troches and the like can contain any of the following ingredients, or compounds of a similar nature: a binder such as microcrystalline cellulose, gum tragacanth or gelatin; an excipient such as starch or lactose, a dispersing agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials that modify the physical form of the dosage unit, for example, coatings of sugar, shellac, or enteric agents. Further, a syrup may contain, in addition to the active compounds, sucrose as a sweetening agent and certain preservatives, dyes, colorings, and flavorings.

The amount of compound administered to the patient is sufficient to radiosensitize the malignant neoplasm to be treated but below that which may elicit toxic effects. This amount will depend upon the type of tumor, the species of the patient being treated, the indication dosage intended and the weight or body surface of the patient. The radiation may be administered to humans in a variety of different fractionation regimes, i.e., the total radiation dose is given in portions over a period of several days to several weeks. These are most likely to vary from daily (i.e., five times per week) doses for up to six weeks, to once weekly doses for four to six weeks.

The amount of radiosensitizing compound administered to the patient may be given prior to radiation treatment, during radiation treatment, or after radiation treatment. However, it is preferred that the compounds of the invention be administered prior to radiation treatment.

After administration of the radiosensitizing composition to the hypoxic tumor cells and the passage of a time interval sufficient to enhance radiosensitization of the hypoxic tumor cells, the hypoxic tumor cells are irradiated with a dose of radiation effective to destroy the hypoxic tumor cells. Generally, the patient will receive a radiation dosage of about 2 Gy per day for five days. Generally, the patient will receive a total radiation dosage of about 70 to about 80 Gy over seven to eight weeks, each individual radiation dose to be given within approximately 1 to 4 hrs after administration of the radiosensitizer. Such sequences of radiosensitization treatments and irradiation are repeated as needed to abate and, optimally, reduce or eliminate, the spread of the malignancy. However, it is understood by one skilled in the art that daily radiation dosage and total radiation dosage will vary depending on a patient’s tumor type, treatment protocol, and physical condition. For example, the daily dose of the present compounds is not specifically limited but can vary with a patient’s age, cancer, body weight, and current treatment protocol and/or medications. Additionally, the present compounds are useful as radiosensitizer and can be administered in one or more doses, i.e. one to several doses, prior to the exposure to radiation.

*Initial Radio-sensitizing Studies Using U87MG Radio-resistant Xenografts in Nude Mice (Non-optimized Dosing Schedule)*

Irradiation of cells induces check point arrest, which allows cells to repair DNA damage, with activated PARP facilitating the repair of DNA damage. Hypothetically, administration of a PARP inhibitor in combination with single dose or fractionated radiation will reduce the ability of irradiated cells to repair DNA damage and increase cell kill. Therefore, a PARP inhibitor should work synergistically with fractionated radiation to increase tumor growth delay. The initial test of this hypothesis with Example 7/Example 6 was conducted in radio-resistant U87MG human glioblastoma xenografts in nude mice. As shown in **Figure 3**, administration of Example 7 alone, radiation alone, and Example 7 in combination with radiation (100 mg/kg dose equivalents of Example 6, s.c. qd two days prior to radiation and in combination with 7.5 Gy radiation for 3 days), was done in mice bearing established tumors. Example 7 administered as a single agent had no effect on tumor growth. Tumors treated with vehicle or Example 7 reached a tumor volume of 2000 mm3 in 10.0 days or 9.6 days ( p = 0.798, vs. control), respectively. Administration of radiation alone increased the time to reach 2000 mm3 to 16.1 days, an increase in tumor growth delay (TGD) of 6.1 days (p=0.033, vs. control). In contrast, administration of Example 7 with radiation therapy increased the time for tumors to reach 2000 mm3 to 24.8 days, corresponding to a 14.8 day TGD. The magnitude of effect with the combination therapy was stronger than that seen by a comparable regimen of Example 7 only (p=0.001), or radiation only (p=0.006) indicating that Example 7 exhibits the profile of a true radio-sensitizer. Plasma levels of Example 6 at Cmax associated with efficacy (at 100 mg/kg Example 7) were 23 μM, comparable to those achieved at this dose in chemo-sensitization studies.

*Radio-sensitizing Studies with Example 7 and a Clinically Relevant Fractionated Radiotherapy Dosing Schedule Using U87MG Radio-resistant Xenografts in Nude Mice*

A subsequent radio-sensitization study was evaluating Example 7 (30 and 100 mg/kg, s.c.) in combination with a clinically-relevant fractionated radiotherapy schedule (2 Gy X 5days). Example 7 was administered 0.5 hr after radiation for 5 days, and dosing of Example 7 continued for 16 days after the radiation regimen was completed. The rationale for this dosing schedule was based on the fact that DNA repair from radiation damage occurs 10-12 days post-radiation, therefore, continual dosing of Example 7 and modulation of PARP activity covers cell cycle arrest and DNA repair time which should act synergistically with fractionated radiation to increase radio-sensitivity and tumor growth delay. As shown in **Figures 1 and 2**, administration of radiation alone (2 Gy X 5days) resulted in a TGD of 2.5 days as compared to vehicle treated tumors. Administration of Example 7 (CEP 30; 30 mg/kg s.c.) increased the TGD to 15 days, a 4 fold increase compared to radiation alone ($p \leq 0.05$); and 26 days, a 6-fold increase compared to Example 7 alone ($p \leq 0.001$). Plasma levels of Example 6 at Cmax associated with radio-sensitization efficacy were 5.5 μM. Administration of Example 7 (100 mg/kg, s.c.) with fractionated radiotherapy resulted in significant anti-tumor efficacy, but 80% mortality by day 11. Plasma levels at Cmax at the 100 mg/kg, s.c. dose were 21 μM, in agreement with exposure levels achieved at this dose in chemo-sensitization studies and the initial radio-sensitization studies described above.

These data demonstrate that a greater increase in TGD was observed at a lower concentration of Example 7 (CEP 30; 30 mg/kg dose equivalents of Example 6 s.c. qd X21 days) using a clinically relevant fractionated dosing schedule. In addition, Example 7 (CEP 30; 30 mg/kg dose equivalents of Example 6 s.c. qd X21 days) alone had no effect on tumor growth inhibition demonstrating that Example 7 acts as a "true" radio-sensitizer.

To evaluate therapeutic gain, Example 7 (30 and 100 mg/kg dose equivalents of Example 6 sc) plus 2 Gy radiation X 5 days was evaluated in bone marrow and jejunal crypt assays to determine if Example 7 potentiated radiation-induced normal tissue (NT) toxicity. Evaluation of bone marrow and intestinal mucosa revealed that Example 7 (30 and 100 mg/kg dose equivalents of Example 6 sc) did not potentiate radiation toxicity in these tissues. studies indicate that CEP-9722 exerts radio-sensitizing effects when administered orally. These combined data indicate that Example 7 acts as a radiosensitizer by increasing the effectiveness of fractionated radiotherapy in a radio-resistant glioma model in a greater than additive manner and does not potentiate radiation-induced NT toxicity.

**Examples**

The compounds of the present invention may be prepared in a number of methods well known to those skilled in the art, including, but not limited to those described below, or through modifications of these methods by applying standard techniques known to those skilled in the art of organic synthesis. All processes disclosed in association with the present invention are contemplated to be practiced on any scale, including milligram, gram, multigram, kilogram, multikilogram or commercial industrial scale.

The present invention features methods for preparing the multicyclic compounds described herein which are useful as inhibitors of PARP. The method consists of a multistep synthesis starting with 4-methoxyindole. Specifically, 4-methoxyindole **A,** is treated serially, for example, with butyllithium, carbon dioxide, t-butyllithium and a ketone **B** to

provide a 2-substituted 4-methoxyindole tertiary alcohol **C.** This tertiary alcohol is eliminated, for example, under acidic conditions using hydrochloric acid or toluenesulfonic acid, to afford a substituted 2-vinylindole, **D.** Diels-Alder cycloaddition of **D** with a dienophile such as, but not limited to, maleimide (**E**) affords the cycloaddition intermediate **F**. Aromatization of the cycloaddition intermediate, for example, with oxygen in the presence of a catalyst such as palladium or platinum or with an oxidant such as DDQ or tetrachloroquinone, produces carbazole **G**.
Further treatment of **G** with an alkylating or acylating reagent gives indole-N-substituted carbazole derivatives of the present invention. Conventional procedures for the selection and preparation of suitable prodrug derivatives are described, for example, in Prodrugs, Sloane, K. B., Ed.; Marcel Dekker: New York, 1992.

The compounds of the present invention are PARP inhibitors. The potency of the inhibitor can be tested by measuring PARP activity *in vitro* or *in vivo.* A preferred assay monitors transfer of radiolabeled ADP-ribose units from $[^{32}P]NAD^+$ to a protein acceptor such as histone or PARP itself. Routine assays for PARP are disclosed in Purnell and Whish, Biochem. J. 1980, 185, 775.

**Example 1**

**Cell Line**

U87MG human glioblastoma cells were cultured in commercially available Minimum Essential Medium (MEM) with 1.5 g/L sodium bicarbonate, 0.1nM non-essential amino acids, 1.0nM sodium pyruvate with 10% Fetal Bovine Serum (FBS).

**Example 2**

**Tumor Cell Implantation and Growth**

Exponentially growing cells were harvested and injected (($2x10^6$) cells/mouse) into the right flank of commercially available athymic NCR NUM nude mice. Animals bearing tumors of 200-400mm$^3$ were randomized according to size into the appropriate treatment groups (n=10). Tumors were measured every 3-4 days using a vernier caliper. Tumor volumes were calculated using the following formula:

$$V(mm^3) = 0.5236 \text{ x length (mm) x width (mm) [length (mm) + width (mm)/2].}$$

**Example 3**

*Methods:* U87MG human glioblastoma cells were injected subcutaneously (s.c.) into the right hind limb of athymic NCR NUM mice and allowed to grow to a mean tumor volume of 200mm$^3$. Mice that received radiotherapy were anesthetized prior to irradiation with 100 mg/kg Ketamine + 10 mg/kg xylezine or 37.5 mg/kg Ketamine + 0.2 mg/kg acepromazine, s.c. to provide 25-30 min of sedation. Anesthetized mice were positioned in malleable lead shielding which conforms to the animal's body size and shape without undue pressure. The body was shielded by lead. The tumor bearing leg or exposed tumor was irradiated with the appropriate dose. After tumors were irradiated, the mice are returned to cages on heating pads until recovered from the anesthetics. Example 7 was given as soon possible (within 30 min) after radiation (RT). **Figure 3**: Mice were randomized into the following treatment groups (n=10): 1) vehicle, 2) radiation only (7.5 Gy for 3 days), 3) Example 7 only (100 mg/kg dose equivalents of Example 6, s.c., QD for 5 days), and 4) Example 7 plus radiation. Either the Example 7 or vehicle was administered s.c. on days 1-5 and 30 minutes after radiation on day 2, 3, and 4. Analysis of data was performed using mixed effects regression to model the base-10 logarithm of tumor volume as a function of time and treatment. Analyses were performed in SAS 8.3 (SAS Institute Inc., Cary, NC). **Figures 1&2**: Mice were randomized into the following treatment groups and administered: 1) vehicle , 2) RT (5 X 2 Gy), 3) RT plus Example 7 (30 or 100 mg/kg s.c. dose equivalents of Example 6, qd X21d) or 4) Example 7 (30 or 100 mg/kg dose equivalents of Example 6 s.c., qd,X21d) only. Example 7 was given on days 1-21 and RT was given on days 1-5. All of the animals were measured on the same day. Individual tumor volume measurements were modeled in a log transformed linear model and the best fit time for tumors to reach approximately 2000 mm$^3$ was determined. One-way ANOVA and post hoc analysis was used to determine significance. A P value $\leq 0.05$ was considered significant.

*Results:* All groups started treatment with similar-sized tumors of 200mm$^3$ (P=0.83 comparing groups at day 0). As shown in **Figure 3**, administration of Example 7 alone, radiation alone, and Example 7 in combination with radiation (100 mg/kg dose equivalents of Example 6, s.c. qd two days prior to radiation and in combination with 7.5 Gy radiation

for 3 days), was done in mice bearing established tumors. Example 7 administered as a single agent had no effect on tumor growth. Tumors treated with vehicle or Example 7 only reached a tumor volume of 2000 $mm^3$ in 10.0 days or 9.6 days ( P = 0.798, vs. control), respectively. Administration of radiation alone increased the time to reach 2000 $mm^3$ to 16.1 days, an increase in tumor growth delay (TGD) of 6.1 days (P=0.033, vs. control). The combination therapy of Example 7 with radiation therapy increased the time for tumors to reach 2000 $mm^3$ to 24.8 days, corresponding to a 14.8 day TGD. The magnitude of effect with the combination therapy was stronger than that seen by a comparable regimen of Example 7 only (P=0.001), or radiation only (P=0.006) indicating that Example 7 exhibits the profile of a true radio-sensitizer. As shown in **Figures 1&2**, administration of Example 7(CEP 30; 30 mg/kg dose equivalents of Example 6 s.c.) in combination with RT increased the TGD to 15 days, a 4 fold increase compared to radiation alone ($P \leq 0.05$); and 26 days, a 6-fold increase compared to Example 7 alone ($P \leq 0.001$). Administration of Example 7 (100 mg/kg, s.c.) with fractionated radiotherapy resulted in significant anti-tumor efficacy, but 80% mortality by day 11. These data demonstrate that a greater increase in TGD was observed at a lower concentration of Example 7 (CEP 30; 30 mg/kg) using a clinically relevant fractionated dosing schedule. In addition, administration of Example 7 alone had no effect on tumor growth inhibition demonstrating that Example 7 acts as a "true" radio-sensitizer.

**Example 4**

**Evaluation of DNA Damage**

Antibodies: Primary antibodies can be used against phospho histone H2AX (Cell Signaling, #2577, 1:1000) and GAPDH (Abcam, #9484, 1:5000). Secondary antibodies can be Goat anti-mouse IRDye800 (Rockland, #610-132-121) and Goat anti-rabbit Alexa fluor 700 (Molecular Probes, #A21038).

U87MG cells can be irradiated with 3Gy or 5Gy radiation, followed by treatment with Example 6 (300 nM and 1 μM) 0.5 hs post-radiation. Samples can then be collected at 0.5, 1, and 4 hours after the addition of Example 6. The cells can then be lysed on ice in RIPA buffer (150 mM NaCl, 1% NP-40, 0.5% Sodium deoxycholate, 0.1% SDS, 50 mM Tris pH 8.0) plus inhibitor cocktail (Protease Inhibitor Cocktail Set III, Calbiochem), and 1 mM $Na_3VO_4$ can then be quantitated using the BCA protein assay kit (Pierce #23225). Samples can be resolved by electrophoresis (15 μg protein) using a 4-12% bis tris gel(Novex #NP0336) with MES SDS buffer (Novex, #NP0002) at 140 volts, and then transferred to a nitrocellulose membrane (Biorad, #162-0145) by semi-dry transfer (18 volts for 35 minutes) using 2X transfer buffer (Novex, #NP0006). Membranes can then be blocked for 1 hour at room temperature in Odyssey Blocking Buffer (Licor # 927-40000) diluted 1:1 with 1X TBS and then incubated overnight at 4°C with both primary antibodies in Odyssey Blocking Buffer diluted 1:1 with 1X TBS-T 0.05%. The next day, membranes can be washed four times with 1X TBS-T 0.2% for 10 minutes each wash, and then incubated with both secondary antibodies at 1:10,000 (in Odyssey Blocking Buffer diluted 1:1 with 1X TBS-T 0.05%) for 1.5 hours at room temperature protected from light. Blots can be washed four times with 1X TBS-T 0.2% for 10 minutes each wash (protected from light) and then read on the Odyssey Infrared Imager. GAPDH can be visualized using the 800nm signal and the phospho-H2AX then detected with 700nm. Size expected for phospho histone H2AX is 15kDa and GAPDH is 36kDa.

**Example 5**

**Cell Cycle Analysis**

U87MG cells can be irradiated at 3Gy or 5Gy radiation and then treated with Example 6 (300 nM and 1 μM) 0.5 hours post-radiation. Samples can then be collected 8, 24, and 48 hours (or any times determined by one skilled in the art) after the addition of Example 6. Cells can be fixed in 100% ethanol overnight at 4°C. The next day cells can be incubated with cell cycle reagent (Guava Technologies #4500-0220) for 1 hour at room temperature protected from light. Stained nuclei are analyzable by flow cytometry (Guava EasyCyte; using settings known to one skilled in the art, for example 427 X8; acquisition data 5,000 events/sample). The percentage of cells in each phase of the cell cycle can be determined using Cell Cycle analysis software (Guava Technologies).

**Example 6**

**7-Methoxy-1,2,3,11-tetrahydro-5,11-diaza-benzo[a]trindene-4,6-dione**

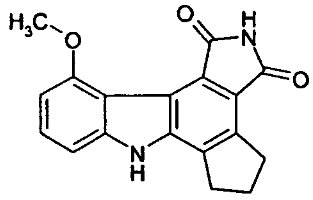

6

Step 1: To a cooled (-78 °C) solution of 4-methoxyindole (2.0 g, 13.1 mmol) in dry THF (20 mL) was slowly added nBuLi in hexanes (2.5 M, 5.2 mL, 13.1 mmol). The mixture was stirred at -78 °C for another 30 min, and $CO_2$ gas was then bubbled into the reaction mixture for 15 min, followed by additional stirring of 15 min. Excess $CO_2$ and half the THF volume was removed at reduced pressure. Additional dry THF (10 mL) was added to the reaction mixture that was cooled back to -78 °C. 1.7 M t-BuLi (7.7 mL, 13.1 mmol) was slowly added to the reaction mixture over 30 min. Stirring was continued for 2 h at -78 °C, followed by slow addition of a solution of cyclopentanone (1.7 g, 20.4 mmol) in dry THF (5 mL). After an additional stirring of 1h at -78 °C, the reaction mixture was quenched by dropwise addition of water (5 mL) followed by saturated $NH_4Cl$ solution (20 mL). Ethyl ether (50 mL) was added and the mixture was stirred for 10 min at room temperature. The organic layer was separated, dried ($MgSO_4$) and concentrated to give a mixture of alcohol (1-(4-methoxy-1H-indol-2-yl)-cyclopentanol) and diene (2-cyclopent-1-enyl-4-methoxy-1H-indole). To the mixture in acetone (15 mL) was added 2 N HCl (5 mL). The mixture was stirred for another 10 min, water (50 mL) was added and the diene product 2-cyclopent-1-enyl-4-methoxy-1H-indole collected and dried under vacuum. The product was purified by silica gel chromatography (EtOAC/hexanes 9:1). $^1H$ NMR (DMSO-d6) δ 1.9-2.1 (m, 3 H), 2.6-2.75 (m, 3H), 3.9 (s, 3H), 6.1 (s, 1H), 6.3 (s, 1H), 6.4 (m, 1H), 6.9-7.0 (m, 2H), 11.1 (s, 1H). This product was used directly in the next step.

Step 2: A mixture of 2-cyclopent-1-enyl-4-methoxy-1H-indole (0.1 g, 0.47 mmol) and maleimide (0.0.9 g, 0.91 mmol) in acetic acid (5 mL) were stirred for 1 hour at room temperature. Water was added and the product extracted with EtOAc, which was washed with 2 N $Na_2CO_3$ solution, water, and saturated NaCl solution and dried ($MgSO_4$). The drying agent was removed by filtration and the solvent concentrated to give 0.13 g MS: m/z 309 (M - H).

Step 3: The product from step 2 (0.123 g, 0.4 mmol) in a toluene (2 mL) and acetic acid (3 mL) was added 2,3-dichloro-5,6-dicyano-1,4-benzoquinone (DDQ, 185 mg, 0.8 mmol). After stirring 30 min at 0 °C the mixture was concentrated and treated with EtOAC and ascorbic acid. After 30 min the mixture was made basic with 2 N $Na_2CO_3$. The EtOAc layer was washed with water, saturated NaCl solution, dried ($MgSO_4$) and concentrated to give the product 0.095 mg; MS: m/z 305 $(M - H)^+$. $^1H$ NMR (DMSO-d6) δ 2.26-2.31 (m, 2H), 3.1-3.2 (m, 2H), 3.3-3.4 (m, 2H), 3.9 (s, 3H), 6.7 (m, 1H), 7.1 (m 1H), 6.4 (m, 1H), 7.4 (m, 1H), 10.6 (s, 1H), 11.9 (s, 1H).

**Example 7**

**7-Methoxy-5-(4-methyl-piperazin-1-ylmethyl)-1,2,3,11-tetrahydro-5,11-diaza-benzo[a]trindene-4,6-dione**

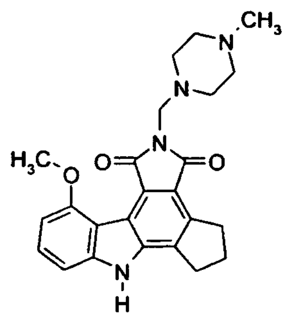

7

To a slurry of Example 6 (10.0 g, 30 mmol) and N-methylpiperazine (12.4 g, 124 mmol) in ethanol (950 mL) was added paraformaldehyde (5.60 g, 62.4 mmol) in 0.5 hr and stirred 24 hr. The slurry was evaporated to dryness. To

the residue was added hexane (500 mL), sonicated 15 min., stirred 1.5 hr. and cooled at 0 °C for 15 min. A yellow solid was collected and washed with cold hexane. This product was dissolved in warm tetrahydrofuran (THF) (250 mL) and filtered. The filtrate was added dropwise into hexane (3 L), stirred 15 min., and Example 7 collected the precipitate and washed with hexane (12.0 g, 96% yield). $^1$H NMR (DMSO-$d_6$) 2.12 (s,3H), 2.35 (m,8H), 2.53 (m,4H), 3.18 (m,2H), 4.44 (s,3H), 6.70 (d,1H), 7.10 (d,1H), 7.40 (t,1H), 11.96 (s,1H), MS m/z 419 (M + H).

**Example 8**

**7-Methoxy-5-(diethylaminomethyl)-1,2,3,11-tetrahydro-5,11-diaza-benzo[a]trindene-4,6-dione (Ex.8a)**

**7-Methoxy-5,11-(bis-diethylaminomethyl)-1,2,3,11-tetrahydro-5,11-diaza-benzo[a]trindene-4,6-dione (Ex.8b)**

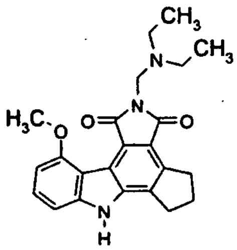

8a

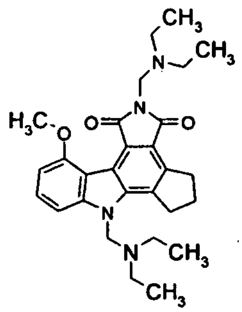

8b

To a slurry of Example 6 (50 mg, 0.16 mmol) in DMF (5 mL) was added paraformaldehyde (73 mg, 0.81 mmol), diethylamine (84 μL, 0.81 mmol) and stirred at room temperature for 1 day. The reaction was evaporated and the residue triturated with hexane and evaporated to give two products as an oil, (ratio 6-1, 16b:16c). $^1$H-NMR (DMSO-$d_6$) 0.98 (t, 3H), 1.11 (t,3H), 2.27 (m,2H), 2.53 (m,8H), 2.57 (m,15H), 3.17 (t,2H), 3.50 (m,1H), 3.97 (s,3H), 4.14 (d,2H), 4.71 (d, 2H), 6.82 (t,2H), 6.75 (d,2H), 7.13 (d,2H), 7.33 (m,1H), 7.46 (t,3H),7.52 (m,1H), 11.95 (s,1H).16b: MS m/z 392. 16c MS m/z 476.

**Example 9**

**7-Methoxy-5,11-(bis-morpholin-4-ylmethyl)-1,2,3,11-tetrahydro-5,11-diaza-benzo[a]trindene-4,6-dione**

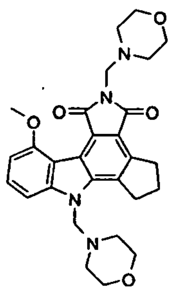

9

To a slurry of Example 6 (15 mg, 0.049 mmol) in DMF (1 mL) was added paraformaldehyde (42 mg, 0.05 μL), morpholine (160 mg, 1.9 mmol) and heated at 70 °C for 18 hr. The mixture was evaporated. The residue was triturated

with hexane, then dissolved in $CH_2Cl_2$, filtered and evaporated. The residue was triturated with $Et_2O$ and Example 9 collected as a yellow solid (5 mg, 20%), $^1$HNMR (DMSO-$d_6$) 7.52 (t, 1H), 7.39 (d, 1H), 6.82 (d, 1H), 5.0 (s, 2H), 4.46 (s, 2H), 3.98 (s, 3H), 3.56 (s, 6H), 3.49 (s, 4H), 2.50 (s, 6H), 2.49 (s, 4H), 2.45 (m, 2H); MS m/z 505 (M + H).

**Example 10**

**7-Methoxy-5-(morpholin-4-ylmethyl)-1,2,3,11-tetrahydro-5,11-diaza-benzo[a]trindene-4,6-dione**

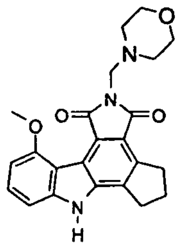

**10**

To a slurry of Example 6 (50 mg,0.16 mmol) in ethanol (10 mL) was added paraformaldehyde (72 mg, 0.8 mmol), morpholine (100 g, 1.1 mol) and heated at 50 °C for 5 hr. The reaction was evaporated, water added (15 mL) and a yellow solid collected (59 mg). $^1$H NMR (DMSO-$d_6$) 11.98 (s, 1H), 7.45 (t, 1H), 7.13 (d, 1H), 6.75 (d, 1H), 4.44 (s, 2H),3.97 (s, 3H), 3.56 (s, 4h),3.18 (t, 2h), 2.29 (t, 2h). MS m/z 406 (M + H).

**Example 11**

**Measurement of PARP Enzymatic Activity.**

PARP activity was monitored by transfer of radiolabeled ADP-ribose units from [$^{32}$P]NAD$^+$ to a protein acceptor such as histone or PARP itself. The assay mixtures contained 100 mM Tris (pH 8.0), 2 mM DTT, 10 mM $MgCl_2$, 20 ug/ml DNA (nicked by sonication), 20 mg/ml histone H1, 5 ng recombinant human PARP, and inhibitor or DMSO (< 2.5% (v/v)) in a final volume of 100 uL. The reactions were initiated by the addition of 100 $\mu$M NAD$^+$ supplemented with 2 uCi [$^{32}$P] NAD$^+$/mL and maintained at room temperature for 12 minutes. Assays were terminated by the addition of 100 $\mu$M of 50% TCA and the radiolabeled precipitate was collected on a 96-well filter plate (Millipore, MADP NOB 50), washed with 25% TCA. The amount of acid-insoluble radioactivity, corresponding to polyADP-ribosylated protein, was quantitated in a Wallac MicroBeta scintillation counter.

**Determination of $IC_{50}$ for Inhibitors.**

Single-point inhibition data were calculated by comparing PARP, VEGFR2, or MLK3 activity in the presence of inhibitor to activity in the presence of DMSO only. Inhibition curves for compounds were generated by plotting percent inhibition versus $\log_{10}$ of the concentration of compound. $IC_{50}$ values were calculated by nonlinear regression using the sigmoidal dose-response (variable slope) equation in GraphPad Prism as follows:

$$y = \text{bottom} + (\text{top} - \text{bottom})/(1 + 10^{(\log IC_{50} - x)*\text{Hillslope}})$$

where y is the % activity at a given concentration of compound, x is the logarithm of the concentration of compound, bottom is the % inhibition at the lowest compound concentration tested, and top is the % inhibition at the highest compound concentration examined. The values for bottom and top were fixed at 0 and 100, respectively. $IC_{50}$ values are reported as the average of at least three separate determinations.

Using the assays disclosed herein the following Table 2 demonstrates the utility of compounds of the invention for PARP inhibition. Compounds of the present invention are considered active if their $IC_{50}$ values are less than 50 uM.

In the following Table, for the inhibition of PARP, compounds of the present invention with a "+" are less than 10000 nM; compounds of the present invention with a "++" are less than 1000 nM; and compounds of the present invention with a "+++" are less than 100 nM in $IC_{50}$ for PARP inhibition. Where no $IC_{50}$ value is represented, data has yet to be determined.

**Table 2**

| Example | No. | PARP $IC_{50}$ (nM) |
|---|---|---|
| 6 | 6 | +++ |
| 7 | 7 | +++ |
| 8 | 8a/8b | +++ |
| 9 | 9 | +++ |
| 10 | 10 | +++ |

**Example 12**

A preliminary study was conducted to determine the radio-sensitizing ability of orally administered Example 7.

*Tumor Cell Implantation and Growth*

Exponentially growing cells were harvested and injected ($2x10^6$ cells/mouse) into the right flank of commercially available athymic NCR nu/nu nude mice. Animals bearing tumors of 200-400mm$^3$ were randomized according to size into the appropriate treatment groups (n=4). Tumors were measured every 3-4 days using a vernier caliper. Tumor volumes were calculated using the following formula:

$$V=a^2b/2$$, where a and b are the short and long dimensions, respectively.

*Methods:* U87MG human glioblastoma cells were injected subcutaneously (s.c.) into the right hind limb of athymic NCR nu/nu nude mice and allowed to grow to a mean tumor volume of 200mm$^3$. Mice that received radiotherapy were anesthetized prior to irradiation with 100 mg/kg Ketamine + 10 mg/kg xylezine or 37.5 mg/kg Ketamine + 0.2 mg/kg acepromazine, s.c. to provide 25-30 min of sedation. Anesthetized mice were positioned in malleable lead shielding which conforms to the animal's body size and shape without undue pressure. The body was shielded by lead. The tumor bearing leg or exposed tumor was irradiated with the appropriate dose. After tumors were irradiated, the mice are returned to cages on heating pads until recovered from the anesthetics. Example 7 was given as soon possible (within 30 min) after radiation (RT). Mice were randomized into the following treatment groups and administered: 1) vehicle , 2) RT (2 Gy X5d), 3) RT plus Example 7 (200 or 300 mg/kg p.o. dose equivalents of Example 6, qd X21d) or 4) Example 7 (200 or 300 mg/kg dose equivalents of Example 6 p.o., qd X21d) only. Example 7 was given on days 1-21 and RT was given on days 1-5. All of the animals were measured on the same day. Individual tumor volume measurements were modeled in a log transformed linear model and the best fit time for tumors to reach approximately 2000 mm$^3$ was determined.

*Results:* As shown in **Figure 5**, administration of Example 7 ( Cep 300; 300 mg/kg dose equivalents of Example 6 p.o. qdX21 d) plus RT (2 Gy X5d) resulted tumor growth stasis starting on day 8 and continuing throughout the study (day 31), while administration of Example 7 (Cep 200; 200 mg/kg dose equivalents of Example 6 p.o. qdX21 d) plus RT (2 Gy X5d) and Example 7 alone (200 and 300 mg/kg dose equivalents of Example 6 p.o. qdX21 d) had no effect on tumor growth as compared to RT alone. The obtained indicating that Example 7 administration only had no effect on tumor growth confirms data obtained from s.c. dosing.

Those skilled in the art will appreciate that numerous changes and modifications can be made to the preferred embodiments of the invention and that such changes and modifications can be made without departing from the spirit of the invention. It is, therefore, intended that the appended claims cover all such equivalent variations as fall within the true spirit and scope of the invention.

**REFERENCES**

Curtin, NJ. (2005) PARP inhibitors for cancer therapy Expert Rev Molec Med 4: 1-20.

Griffin, R et al. (1998) Resistance-modifying agents. 5. Synthesis and biological properties of quinazolinone inhibitors of the DNA repair enzyme poly(ADP-ribose)polymerase (PARP) J. Med. Chem., 42: 5247-5256.

Bowman, KJ, et al. (1998) Potentiation of anti-cancer agent cytotoxicity by the potent poly(ADP-ribose)polymerase inhibitors NU 1025 and NU 1064 Br. J, Cancer 78: 1269-1277.

Bowman, KJ, et al (2001) Differential effects of the poly(ADP-ribose)polymerase inhibitor NU 1025 on topoisomerase I and II inhibitor cytotoxicity in L1210 cells in vitro Br. J. Cancer 84: 106-112.

Chen & Pan (1988) Potentiation of antitumor activity of cisplatin in mice by 3-aminobenzamide and nictotinamide Cancer Chemoth. Pharmacol. 22: 303-307.

Delaney, CA et al. (2000) Potentiation of temozolomide and topotecan growth inhibition and cytotoxicity by novel poly(adenosine diphosphoribose)polymerase inhibitors in a panel of human tumor lines Clin. Cancer Res. 6: 2860-2867.

Griffin R et al., (1995) The role of inhibitors of poly(ADP-ribose)polymerase as resistance-modifying agents in cancer therapy Biochimie 77: 408-422.

Liu, L, et al. (1999) Pharmacologic disruption of base excision repair sensitizes mismatch repair-deficient and-proficient colon cancer cells to methylating agents Clin. Cancer Res. 5: 2908-2917.

Tentori, L. et al. (2002) Combined treatment with temozolomide and poly(ADP-ribose) polymerase inhibitor enhances survival of mice bearing hematologic malignancy at the central nervous system site Blood 15: 2241-2244.

Baldwin J. (2002) FDA evaluating oxaliplatin for advance colorectal cancer treatment J. Natl. Cancer Inst. 94: 1191-1193, 2002.

Jagtap P and Szabo C (2005). Poly (ADP-Ribose) polymerase and the therapeutic effects of its inhibitors. Nature Rev Drug Disc 4: 421-440.

Ame JC, et al (2004). The PARP superfamily. Bioessays 26: 882-893.

Smith, S. (2001) The World According to PARP Trends Biochm.Sci. 26: 174-179.

Virag, L. and Szabo, C. (2002) The therapeutic potential of poly(ADP-ribose) polymerase inhibitors Pharmacol. Rev. 54: 375-429.

de Murcia, JM, et al. (1997) Requirement of poly(ADP-ribose)polymerase in recovery from DNA damage in mice and cells Proc Natl Acad Sci USA 94: 7303-7307.

Masuntani, M, et al. (2000) The response of PARP knockout mice against DNA damaging agents Mutat. Res. 462: 159-166.

Kato, T et al. (1988) Enhancement of bleomycin activity by 3-aminobenzamide, a poly(ADP-ribose) synthesis inhibitor, in vitro and in vivo Anticancer Res. 8: 239-244.

Smith, L. et al. (2005) The novel poly(ADP-Ribose) polymerase inhibitor, AG14361, sensitizes cells to topoisomerase I poisons by increasing the persistence of DNA strand breaks Cl Cancer Res. 11: 8449-8457.

## Claims

1. A pharmaceutical composition for radiosensitizing cancer cells comprising a radiosensitizing amount of a compound of Formula (I):

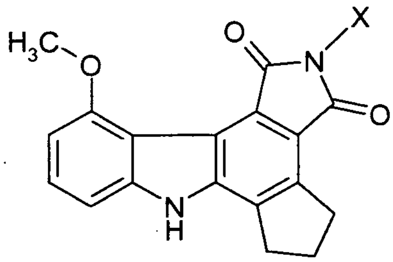

(I)

or a pharmaceutically acceptable salt form thereof, wherein X is H or a prodrug moiety; and a pharmaceutically acceptable carrier.

**2.** The pharmaceutical composition of Claim 1 wherein the prodrug moiety is selected from the group consisting of $-CH_2NR^1R^2$, $CH_2OC(=O)R^3$, $-CH_2OP(=O)(OH)_2$, and $-C(=O)R^4$;

$R^1$ is H or $C_{1-4}$ alkyl;
$R^2$ is H or $C_{1-4}$ alkyl;
alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$ alkyl;
$R^3$ is selected from the group consisting of $-C_{1-4}$ alkyl-$NR^1R^2$, $-C_{1-4}$ alkyl-$OR^5$, pyridinyl, -phenyl($CH_2NR^1R^2$), and $-CH(R^6)NH_2$;
$R^4$ is selected from the group consisting of -O-($C_{1-4}$ alkyl)-$NR^1R^2$, -O-($C_{1-4}$ alkyl)- $OR^5$, and $-CH(R^6)NH_2$;
$R^5$ is H or $C_{1-4}$ alkyl; and
$R^6$ is the side chain of a naturally occurring amino acid.

**3.** The pharmaceutical composition of Claim 1 wherein the prodrug moiety is $-CH_2NR^1R^2$,

$R^1$ is H or $C_{1-4}$ alkyl;
$R^2$ is H or $C_{1-4}$ alkyl; and
alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$ alkyl.

**4.** A pharmaceutical composition as set forth in Claim 1 wherein the compound is

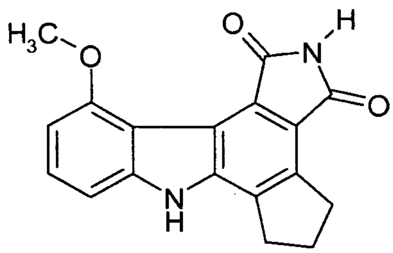

or a pharmaceutically acceptable salt form thereof.

**5.** A pharmaceutical composition as set forth in Claim 1 wherein the compound is

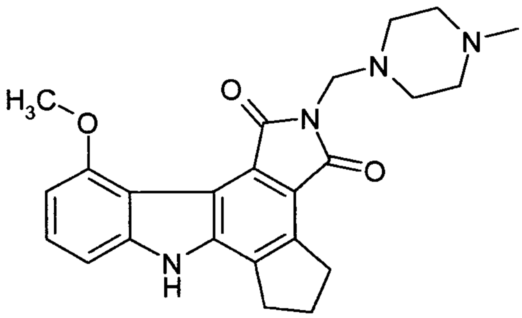

or a pharmaceutically acceptable salt form thereof.

**6.** A compound of Formula (II):

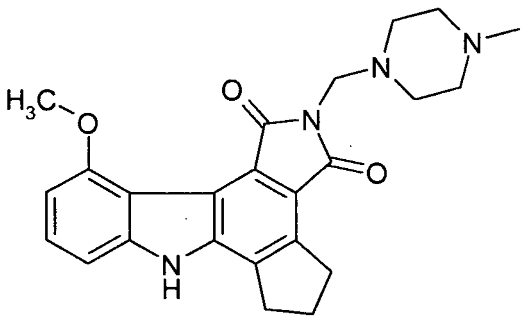

(II)

or a pharmaceutically acceptable salt form thereof.

**7.** A radiosensitizing agent of Formula (I):

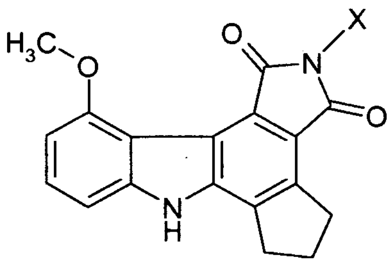

(I)

or a pharmaceutically acceptable salt form thereof, wherein, X is H or a prodrug moiety; for use in the treatment of cancer in a mammal by administering said radiosensitizing agent and applying ionizing radiation to said mammal tissue.

**8.** The radiosensitizing agent of Claim 7 wherein said radiosensitizing agent is present within or proximate to said tissue increases the efficiency of conversion of said applied ionizing radiation into localized therapeutic effects.

**9.** The radiosensitizing agent of Claim 8 wherein said radiosensitizing agent is present in an amount effective to radiosensitize cancer cells.

**10.** The radiosensitizing agent of Claim 9 wherein ionizing radiation of said tissue is performed with a dose of radiation effective to destroy said cells.

**11.** The radiosensitizing agent of Claim 10 wherein said ionizing radiation is of clinically acceptable or recommended radiotheraputic protocols for a given cancer type.

**12.** The radiosensitizing agent of Claim 10 wherein said cancer is malignant.

**13.** The radiosensitizing agent of Claim 10 wherein said cancer is benign.

**14.** The radiosensitizing agent of Claim 7 wherein the prodrug moiety is selected from the group consisting of $-CH_2NR^1R^2$, $-CH_2OC(=O)R^3$, $-CH_2OP(=O)(OH)_2$, and $-C(=O)R^4$;
wherein:

$R^1$ is H or $C_{1-4}$ alkyl;
$R^2$ is H or $C_{1-4}$ alkyl; alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$ alkyl;
$R^3$ is selected from the group consisting of $-C_{1-4}$ alkyl-$NR^1R^2$, $-C_{1-4}$ alkyl-$OR^5$, pyridinyl, -phenyl($CH_2NR^1R^2$), and $-CH(R^6)NH_2$;
$R^4$ is selected from the group consisting of -O-($C_{1-4}$ alkyl)-$NR^1R^2$, -O-($C_{1-4}$ alkyl)- $OR^5$, and $-CH(R^6)NH_2$;
$R^5$ is H or $C_{1-4}$ alkyl; and
$R^6$ is the side chain of a naturally occurring amino acid.

**15.** The radiosensitizing agent of Claim 7 wherein the prodrug moiety is $-CH_2NR^1R^2$,

$R^1$ is H or $C_{1-4}$ alkyl;
$R^2$ is H or $C_{1-4}$ alkyl; and
alternatively, $R^1$ and $R^2$, together with the nitrogen atom to which they are attached, form a heterocyclyl group selected from pyrrolyl, pyrrolidinyl, piperidinyl, morpholinyl, thiomorpholinyl, and piperazinyl, wherein said heterocyclyl group is optionally substituted with $C_{1-4}$ alkyl.

**16.** The radiosensitizing agent of Claim 7 wherein the prodrug moiety is a Mannich base.

**17.** The radiosensitizing agent of Claim 14 wherein the Mannich base is selected form 4-methyl-piperazin-1-ylmethyl-, morpholin-4-ylmethyl-, and 5-diethylaminomethyl-.

**18.** The radiosensitizing agent of Claim 14 wherein the Mannich base is 4-methyl-piperazin-1-ylmethyl.

**19.** A radiosensitizing agent of Claim 7 wherein the route of administration is intravenous, subcutaneous, oral or intraperitoneally.

**20.** A radiosensitizing agent of Claim 7 wherein the route of administration is intravenous.

**21.** A radiosensitizing agent according to Claim 7 wherein said cancer is selected from head and neck squamous cell carcinoma (eye, lip, oral , pharynx, larynx, nasal, carcinoma of the tongue, and esophogeal carcinoma), melanoma, squamous cell carcinoma (epidermis), glioblastoma, astrocytoma, oligodendroglioma, oligoastrocytoma, meningioma, neuroblastoma, rhabdomyosarcoma, soft-tissue sarcomas, osteosarcoma, hematologic malignancy at the cns site, breast carcinoma (ductal and carcinoma in situ), thyroid carcinoma (papillary and follicular), lung carcinoma (bronchioloalveolar carcinoma, small cell lung carcinoma, mixed small cell/large cell carcinoma, combined small cell carcinoma, non-small cell lung carcinoma, squamous cell carcinoma, large cell carcinoma, and adenocarcinoma of the lung), hepatocellular carcinoma, colo-rectal carcinoma, cervical carcinoma, ovarian carcinoma, prostatic carcinoma, testicular carcinoma, gastric carcinoma, pancreatic carcinoma, cholangiosarcoma, lymphoma (Hodgkins and non-Hodgkins types of T-and B-cell origin), leukemia (acute and chronic leukemias of myeloid and lymphoid origins), and bladder carcinoma.

**22.** A radiosensitizing agent according to Claim 7 wherein said cancer is selected from head and neck squamous cell carcinoma (eye, lip, oral, pharynx, larynx, nasal, carcinoma of the tongue, and esophogeal carcinoma), melanoma,

squamous cell carcinoma (epidermis), glioblastoma, neuroblastoma, rhabdomyosarcoma, lung carcinoma, (bronchioloalveolar carcinoma, small cell lung carcinoma, mixed small cell/large cell carcinoma, combined small cell carcinoma, non-small cell lung carcinoma, squamous cell carcinoma, large cell carcinoma, and adenocarcinoma of the lung), lymphoma (Hodgkins and non-Hodgkins types of T-and B-cell origin), and leukemia (acute and chronic leukemias of myeloid and lymphoid origins).

**23.** A radiosensitizing agent according to Claim 7 of formula 7-methoxy-1,2,3,11-tetrahydro-5,11-diaza-benzo[a]trindene-4,6-dione, or a pharmaceutically acceptable salt form thereof.

**24.** A radiosensitizing agent according to Claim 15 of formula 7-methoxy-5-(4-methyl-piperazin-1-ylmethyl)-1,2,3,11-tetrahydro-5,11-diaza-benzo[a]trindene-4,6-dione, or a pharmaceutically acceptable salt form thereof.

**Patentansprüche**

**1.** Pharmazeutische Zusammensetzung zur Radiosensibilisierung von Krebszellen, enthaltend eine radiosensibilisierende Menge einer Verbindung der Formel (I):

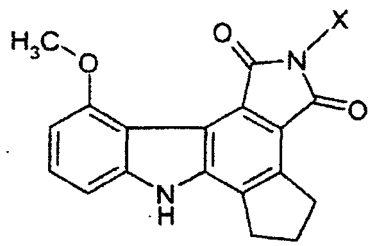

(I)

oder einer pharmazeutisch unbedenklichen Salzform davon, wobei X für H oder eine Prodrug-Gruppe steht; und einen pharmazeutisch unbedenklichen Träger.

**2.** Pharmazeutische Zusammensetzung nach Anspruch 1, wobei die Prodrug-Gruppe ausgewählt ist aus der Gruppe bestehend aus $-CH_2NR^1R^2$, $-CH2OC(=O)R^3$, $-CH_2OP(=O)(OH)_2$ und $-C(=O)R^4$;

$R^1$ für H oder $C_{1-4}$-Alkyl steht;
$R^2$ für H oder $C_{1-4}$-Alkyl steht;
$^1$ und $R^2$ R alternativ dazu zusammen mit dem Stickstoffatom, an das sie gebunden sind, eine Heterocyclylgruppe ausgewählt aus Pyrrolyl, Pyrrolidinyl, Piperidinyl, Morpholinyl, Thiomorpholinyl und Piperazinyl bilden, wobei diese Heterocyclylgruppe gegebenenfalls durch $C_{1-4}$-Alkyl substituiert ist;
$R^3$ ausgewählt ist aus der Gruppe bestehend aus $-C_1$- $_4$-Alkyl-$NR^1R^2$, $-C_{1-4}$-Alkyl-$OR^5$, Pyridinyl, -Phenyl-$(CH_2NR^1R^2)$ und $-CH(R^6)NH_2$;
$R^4$ ausgewählt ist aus der Gruppe bestehend aus -O- ($C_{1-4}$-Alkyl)-$NR^1R^2$, -O-($C_{1-4}$-Alkyl)-$OR^5$ und -CH ($R^6$) $NH_2$;
$R^5$ für H oder $C_{1-4}$-Alkyl steht; und
$R^6$ für die Seitenkette einer natürlichen Aminosäure steht.

**3.** Pharmazeutische Zusammensetzung nach Anspruch 1, wobei es sich bei der Prodrug-Gruppe um $-CH_2NR^1R^2$ handelt;

$R^1$ für H oder $C_{1-4}$-Alkyl steht;
$R^2$ für H oder $C_{1-4}$-Alkyl steht; und
$R^1$ und $R^2$ alternativ dazu zusammen mit dem Stickstoffatom an das sie gebunden sind, eine Heterocyclylgruppe ausgewählt aus Pyrrolyl, Pyrrolidinyl, Piperidinyl, Morpholinyl, Thiomorpholinyl und Piperazinyl bilden, wobei diese Heterocyclylgruppe gegebenenfalls durch $C_{1-4}$-Alkyl substituiert ist.

**4.** Pharmazeutische Zusammensetzung nach Anspruch 1, wobei es sich bei der Verbindung um

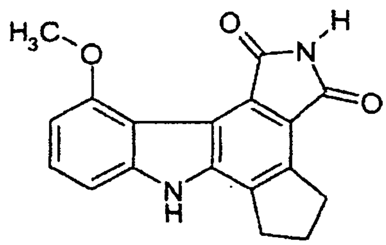

oder eine pharmazeutisch unbedenkliche Salzform davon handelt.

**5.** Pharmazeutische Zusammensetzung nach Anspruch 1, wobei es sich bei der Verbindung um

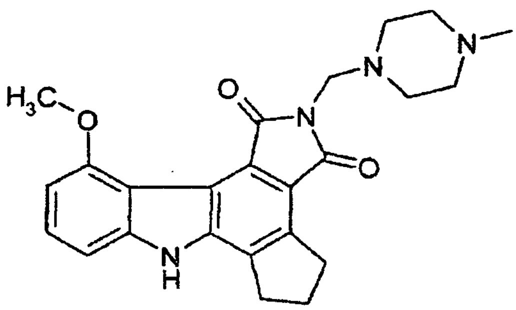

oder eine pharmazeutisch unbedenkliche Salzform davon handelt.

**6.** Verbindung der Formel (II):

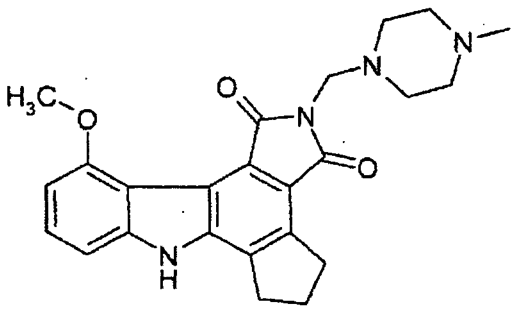

(II)

oder eine pharmazeutisch unbedenkliche Salzform davon.

**7.** Radiosensibilisierendes Mittel der Formel (I):

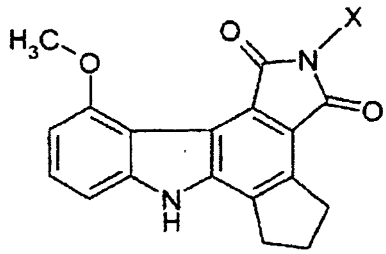

(I)

oder eine pharmazeutisch unbedenkliche Salzform davon, wobei X für H oder eine Prodrug-Gruppe steht; zur Verwendung bei der Behandlung von Krebs in einem Säugetier durch Verabreichung des radiosensibilisierenden Mittels und Anwendung ionisierender Strahlung auf dieses Säugetiergewebe.

**8.** Radiosensibilisierendes Mittel nach Anspruch 7, wobei durch das Vorhandensein des radiosensibilisierenden Mittels in dem Gewebe bzw. in der Nähe des Gewebes die Wirksamkeit der Umwandlung der angewendeten ionisierenden Strahlung in lokalisierte therapeutische Wirkungen erhöht wird.

**9.** Radiosensibilisierendes Mittel nach Anspruch 8, wobei das radiosensibilisierende Mittel in einer für die Radionsensibilisierung von Krebszellen wirksamen Menge vorliegt.

**10.** Radiosensibilisierendes Mittel nach Anspruch 9, wobei die ionisierende Bestrahlung des Gewebes mit einer die Zerstörung dieser Zellen bewirkenden Strahlungsdosis erfolgt.

**11.** Radiosensibilisierendes Mittel nach Anspruch 10, wobei die ionisierende Strahlung klinisch unbedenklichen oder empfohlenen radiotherapeutischen Protokollen für einen gegebenen Krebstyp entspricht.

**12.** Radiosensibilisierendes Mittel nach Anspruch 10, wobei der Krebs bösartig ist.

**13.** Radiosensibilisierendes Mittel nach Anspruch 10, wobei der Krebs gutartig ist.

**14.** Radiosensibilisierendes Mittel nach Anspruch 7, wobei die Prodrug-Gruppe ausgewählt ist aus der Gruppe bestehend aus $-CH_2NR^1R^2$, $-CH_2OC(=O)R^3$, $-CH_2OP(=O)(OH)_2$ und $-C(=O)R^4$ ;
worin

$R^1$ für H oder $C_{1-4}$-Alkyl steht;
$R^2$ für H oder $C_{1-4}$-Alkyl steht;
$R^1$ und $R^2$ alternativ dazu zusammen mit dem Stickstoffatom, an das sie gebunden sind, eine Heterocyclylgruppe ausgewählt aus Pyrrolyl, Pyrrolidinyl, Piperidinyl, Morpholinyl, Thiomorpholinyl und Piperazinyl bilden, wobei diese Heterocyclylgruppe gegebenenfalls durch $C_{1-4}$-Alkyl substituiert ist;
$R^3$ ausgewählt ist aus der Gruppe bestehend aus $-C_{1-4}$-Alkyl-$NR^1R^2$, $-C_{1-4}$-Alkyl-$OR^5$, Pyridinyl, -Phenyl-$(CH_2NR^1R^2)$ und $-CH(R^6)\ NH_2$;
$R^4$ ausgewählt ist aus der Gruppe bestehend aus -O- ($C_{1-4}$-Alkyl)-$NR^1R^2$, -O-($C_{1-4}$-Alkyl)-$OR^5$ und $-CH(R^6)NH_2$;
$R^5$ für H oder $C_{1-4}$-Alkyl steht; und
$R^6$ für die Seitenkette einer natürlichen Aminosäure steht.

**15.** Radiosensibilisierendes Mittel nach Anspruch 7, wobei es sich bei der Prodrug-Gruppe um $-CH_2NR^1R^2$ handelt;

$R^1$ für H oder $C_{1-4}$-Alkyl steht;
$R^2$ für H oder $C_{1-4}$-Alkyl steht; und
$R^1$ und $R^2$ alternativ dazu zusammen mit dem Stickstoffatom an das sie gebunden sind, eine Heterocyclylgruppe ausgewählt aus Pyrrolyl, Pyrrolidinyl, Piperidinyl, Morpholinyl, Thiomorpholinyl und Piperazinyl bilden, wobei diese Heterocyclylgruppe gegebenenfalls durch $C_{1-4}$-Alkyl substituiert ist.

**16.** Radiosensibilisierendes Mittel nach Anspruch 7, wobei es sich bei der Prodrug-Gruppe um eine Mannich-Base

handelt.

**17.** Radiosensibilisierendes Mittel nach Anspruch 14, wobei die Mannich-Base ausgewählt ist aus 4-Methylpiperazin-1-ylmethyl-, Morpholin-4-ylmethyl- und 5-Diethylaminomethyl-.

**18.** Radiosensibilisierendes Mittel nach Anspruch 14, wobei es sich bei der Mannich-Base um 4-Methylpiperazin-1-ylmethyl handelt.

**19.** Radiosensibilisierendes Mittel nach Anspruch 7, wobei die Verabreichungsroute intravenös, subkutan, oral oder intraperitoneal ist.

**20.** Radiosensibilisierendes Mittel nach Anspruch 7, wobei die Verabreichungsroute intravenös ist.

**21.** Radiosensibilisierendes Mittel nach Anspruch 7, wobei der Krebs ausgewählt ist aus Kopf- und Hals-Plattenepitelkarzinom (Auge, Lippe, Mund, Rachen, Kehlkopf, Nase, Zungenkarzinom und Speiseröhrenkarzinom), Melanom, Plattenepitelkarzinom (Epidermis), Glioblastom, Astrozytom, Oligodendrogliom, Oligoastrozytom, Meningiom, Neuroblastom, Rhabdomyosarkom, Weichteilsarkomen, Osteosarkom, hämatologischen Malignitäten des ZNS, Brustkarzinom (duktal und Karzinom in situ), Schilddrüsenkarzinom (papillär und follikulär), Lungenkarzinom (bronchioloalveolares Karzinom, kleinzelliges Lungenkarzinom, gemischtes kleinzelliges/großzelliges Karzinom, kombiniertes kleinzelliges Karzinom, nicht kleinzelliges Lungenkarzinom, Plattenepitelkarzinom, großzelliges Karzinom und Adenokarzinom der Lunge), hepatozelluläres Karzinom, kolorektales Karzinom, Gebärmutterhalskarzinom, Eierstockkarzinom, Prostatakarzinom, Hodenkarzinom, Magenkarzinom, Bauchspeicheldrüsenkarzinom, Cholangiosarkom, Lymphom (Hodgkins- und nicht-Hodgkins-Typen mit T- und B-Zellen-Ursprung), Leukämie (akute und chronische Leukämien myeloiden und lymphoiden Ursprungs) und Blasenkarzinom.

**22.** Radiosensibilisierendes Mittel nach Anspruch 7, wobei der Krebs ausgewählt ist aus Kopf- und Hals-Plattenepitelkarzinom (Auge, Lippe, Mund, Rachen, Kehlkopf, Nase, Zungenkarzinom und Speiseröhrenkarzinom), Melanom, Plattenepitelkarzinom (Epidermis), Glioblastom, Neuroblastom, Rhabdomyosarkom, Lungenkarzinom (bronchioloalveolares Karzinom, kleinzelliges Lungenkarzinom, gemischtes kleinzelliges/großzelliges Karzinom, kombiniertes kleinzelliges Karzinom, nicht kleinzelliges Lungenkarzinom, Plattenepitelkarzinom, großzelliges Karzinom und Adenokarzinom der Lunge), Lymphom (Hodgkins- und nicht-Hodgkins-Typen mit T- und B-Zellen-Ursprung) und Leukämie (akute und chronische Leukämien myeloiden und lymphoiden Ursprungs).

**23.** Radiosensibilisierendes Mittel nach Anspruch 7 mit der Formel 7-Methoxy-1,2,3-11-tetrahydro-5,11-diazabenzo[a]trinden-4,6-dion oder eine pharmazeutisch unbedenkliche Salzform davon.

**24.** Radiosensibilisierendes Mittel nach Anspruch 15 mit der Formel 7-Methoxy-5-(4-methylpiperazin-1-ylmethyl)-1,2,3,11-tetrahydro-5,11-diazabenzo[a]trinden-4,6-dion oder eine pharmazeutisch unbedenkliche Salzform davon.

## Revendications

**1.** Composition pharmaceutique pour la radiosensibilisation des cellules cancéreuses comprenant une quantité radiosensibilisante d'un composé de formule (I) :

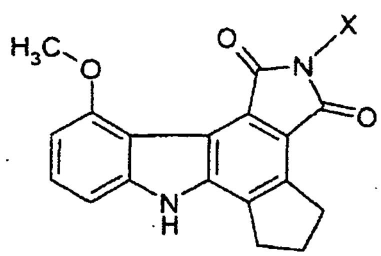

(I)

ou d'une forme de sel pharmaceutiquement acceptable de celui-ci, dans laquelle X est H ou un motif prodrogue ;

et un support pharmaceutiquement acceptable.

**2.** Composition pharmaceutique selon la revendication 1, **caractérisée en ce que** le motif prodrogue est choisi dans le groupe constitué de $-CH_2NR^1R^2$, $-CH_2OC(=O)R^3$, $-CH_2OP(=O)(OH)$ et $-C(=O)R^4$;

$R^1$ est H ou $C_{1-4}$-alkyle ;
$R^2$ est H ou $C_{1-4}$-alkyle ;
selon une alternative, $R^1$ et $R^2$, ensemble avec l'atome d'azote auquel ils sont liés forment un groupement hétérocyclyle choisi parmi pyrrolyle, pyrrolidinyle, pipéridinyle, morpholinyle, thiomorpholinyle, et pipérazinyle, ledit groupement hétérocyclyle étant éventuellement substitué par $C_{1-4}$-alkyle ;
$R^3$ est choisi dans le groupe constitué de $-C_{1-4}$- alkyl-$NR^1R^2$, $-C_{1-4}$-alkyl-$OR^5$, pyridinyle, -phényl $(CH_2NR^1R^2)$ et $-CH(R^6)NH_2$ ;
$R^4$ est choisi dans le groupe constitué de -O-($C_{1-4}$- alkyl)-$NR^1R^2$, -O-($C_{1-4}$-alkyl)-$OR^5$ et $-CH(R^6)NH_2$ ;
$R^5$ est H ou $C_{1-4}$-alkyle ; et
$R^6$ est la chaîne latérale d'un acide aminé naturel.

**3.** Composition pharmaceutique selon la revendication 1, **caractérisée en ce que** le motif prodrogue est $-CH_2NR^1R^2$,

$R^1$ est H ou $C_{1-4}$-alkyle ;
$R^2$ est H ou $C_{1-4}$-alkyle ; et
selon une alternative, $R^1$ et $R^2$, ensemble avec l'atome d'azote auquel ils sont liés forment un groupement hétérocyclyle choisi parmi pyrrolyle, pyrrolidinyle, pipéridinyle, morpholinyle, thiomorpholinyle, et pipérazinyle, ledit groupement hétérocyclyle étant éventuellement substitué par $C_{1-4}$-alkyle.

**4.** Composition pharmaceutique selon la revendication 1 **caractérisée en ce que** le composé est

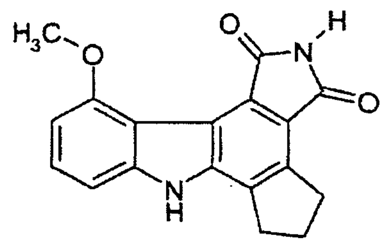

ou une forme de sel pharmaceutiquement acceptable de celui-ci.

**5.** Composition pharmaceutique selon la revendication 1, **caractérisée en ce que** le composé est

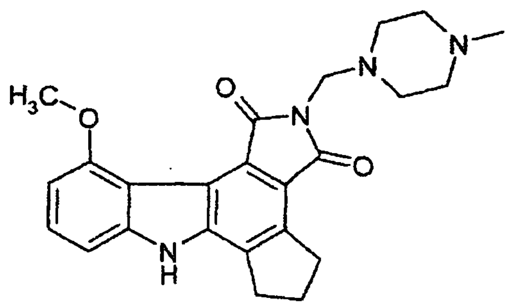

ou une forme de sel pharmaceutiquement acceptable de celui-ci.

**6.** Composé de formule (II):

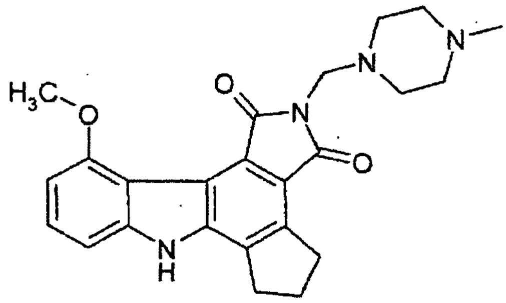

(II)

ou une forme de sel pharmaceutiquement acceptable de celui-ci.

**7.** Agent radiosensibilisant de formule (I) :

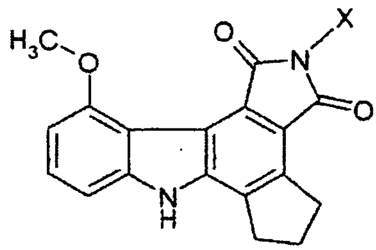

(I)

ou une forme de sel pharmaceutiquement acceptable de celui-ci, dans laquelle X est H ou un motif prodrogue ; destiné à être utilisé dans le traitement du cancer chez un mammifère en administrant ledit agent radiosensibilisant et en appliquant des radiations ionisantes aux tissus dudit mammifère.

**8.** Agent radiosensibilisant selon la revendication 7, **caractérisé en ce que** ledit agent radiosensibilisant est présent au sein ou à proximité dudit tissu et augmente l'efficacité de transformation desdites radiations ionisantes appliquées en effets thérapeutiques localisés.

**9.** Agent radiosensibilisant selon la revendication 8, **caractérisé en ce que** ledit agent radiosensibilisant est présent en une quantité efficace pour radiosensibiliser des cellules cancéreuses.

**10.** Agent radiosensibilisant selon la revendication 9, **caractérisé en ce que** ladite irradiation ionisante dudit tissu est effectuée avec une dose de radiations efficace pour détruire lesdites cellules.

**11.** Agent radiosensibilisant selon la revendication 10, **caractérisé en ce que** ladite irradiation ionisante comprend des protocoles radiothérapeutiques cliniquement acceptables ou recommandés pour un type de cancer donné.

**12.** Agent radiosensibilisant selon la revendication 10, **caractérisé en ce que** le cancer est malin.

**13.** Agent radiosensibilisant selon la revendication 10, **caractérisé en ce que** le cancer est bénin.

**14.** Agent radiosensibilisant selon la revendication 7, **caractérisé en ce que** le motif prodrogue est choisi dans le groupe constitué de $-CH_2NR^1R^2$, $-CH_2OC(=O)R^3$, $-CH_2OP(=O)(OH)_2$ et $-C(=O)R^4$;

$R^1$ est H ou $C_{1-4}$-alkyle ;
$R^2$ est H ou $C_{1-4}$-alkyle ;
selon une alternative, $R^1$ et $R^2$, ensemble avec l'atome d'azote auquel ils sont liés forment un groupement

hétérocyclyle choisi parmi pyrrolyle, pyrrolidinyle, pipéridinyle, morpholinyle, thiomorpholinyle, et pipérazinyle, ledit groupement hétérocyclyle étant éventuellement substitué par $C_{1-4}$-alkyle ;
$R^3$ est choisi dans le groupe constitué de -$C_{1-4}$- alkyl-$NR^1R^2$, -$C_{1-4}$-alkyl-$OR^5$, pyridinyle, -phényl-($CH_2NR^1R^2$) et -$CH(R^6)NH_2$ ;
$R^4$ est choisi dans le groupe constitué de -O-($C_{1-4}$- alkyl) -$NR^1R^2$, -O- ($C_{1-4}$-alkyl) -$OR^5$ et -$CH(R^6)NH_2$ ;
$R^5$ est H ou $C_{1-4}$-alkyle ; et
$R^6$ est la chaîne latérale d'un acide aminé naturel.

**15.** Agent radiosensibilisant selon la revendication 7, **caractérisé en ce que** le motif prodrogue est -$CH_2NR^1R^2$,

$R^1$ est H ou $C_{1-4}$-alkyle ;
$R^2$ est H ou $C_{1-4}$-alkyle ; et

selon une alternative, $R^1$ et $R^2$, ensemble avec l'atome d'azote auquel ils sont liés forment un groupement hétérocyclyle choisi parmi pyrrolyle, pyrrolidinyle, pipéridinyle, morpholinyle, thiomorpholinyle, et pipérazinyle, ledit groupement hétérocyclyle étant éventuellement substitué par $C_{1-4}$-alkyle.

**16.** Agent radiosensibilisant selon la revendication 7, **caractérisé en ce que** le motif prodrogue est une base de Mannich.

**17.** Agent radiosensibilisant selon la revendication 14 **caractérisé en ce que** la base de Mannich est choisie parmi 4-méthylpipérazin-1-ylméthyl-, morpholin-4-ylméthyl- et 5-diéthylaminométhyl-.

**18.** Agent radiosensibilisant selon la revendication 14 **caractérisé en ce que** la base de Mannich est 4-méthylpipérazin-1-ylméthyle.

**19.** Agent radiosensibilisant selon la revendication 7, **caractérisé en ce que** la voie d'administration est la voie intraveineuse, sous-cutanée, orale ou intrapéritonéale.

**20.** Agent radiosensibilisant **caractérisé en ce que** la voie d'administration est la voie intraveineuse.

**21.** Agent radiosensibilisant selon la revendication 7, **caractérisé en ce que** ledit cancer est choisi parmi le carcinome spinocellulaire de la tête et du cou (carcinome de l'oeil, de la lèvre, de la bouche, du pharynx, du larynx, du nez, la langue et carcinome oesophagien), mélanome, carcinome spinocellulaire (épiderme), glioblastome, astrocytome, oligodendrogliome, oligoastrocytome, méningiome, neuroblastome, rhabdomyosarcome, sarcomes des tissus mous, ostéosarcome, malignité hématologique au site du snc, carcinome de sein (canalaire et carcinome in situ), carcinome thyroïdien (papillaire et folliculaire), carcinome pulmonaire (carcinome bronchioloalvéolaire, carcinome pulmonaire à petites cellules, carcinome mixte à petites cellules/grandes cellules, carcinome combiné à petites cellules, carcinome pulmonaire à cellules non petites, carcinome spinocellulaire, carcinome à grandes cellules, et adénocarcinome du poumon), carcinome hépatocellulaire, carcinome colo-rectal, carcinome du col de l'utérus, carcinome ovarien, carcinome prostatique, carcinome testiculaire, carcinome gastrique, carcinome pancréatique, cholangiosarcome, lymphome, (de types Hodgkins et non Hodgkins provenant de cellules T et B), leucémie (leucémies aigües et chroniques d'origines myéloïde et lymphoïde), et carcicome de la vessie.

**22.** Agent radiosensibilisant selon la revendication 7, **caractérisé en ce que** ledit cancer est choisi parmi le carcinome spinocellulaire de la tête et du cou (carcinome de l'oeil, de la lèvre, de la bouche, du pharynx, du larynx, du nez, la langue et carcinome oesophagien), mélanome, carcinome spinocellulaire (épiderme), glioblastome, neuroblastome, rhabdomyosarcome, carcinome pulmonaire (carcinome bronchioloalvéolaire, carcinome pulmonaire à petites cellules, carcinome mixte à petites cellules/grandes cellules, carcinome combiné à petites cellules, carcinome pulmonaire à cellules non petites, carcinome spinocellulaire, carcinome à grandes cellules, et adénocarcinome du poumon), lymphome, (de types Hodgkins et non Hodgkins provenant de cellules T et B), leucémie (leucémies aigües et chroniques d'origines myéloïde et lymphoïde).

**23.** Agent radiosensibilisant selon la revendication 7, de formule 7-méthoxy-1,2,3-11-tétrahydro-5,11-diazabenzo[a]trindène-4,6-dione, ou d'une forme de sel pharmaceutiquement acceptable de celle-ci.

**24.** Agent radiosensibilisant selon la revendication 15 de formule 7-méthoxy-5-(4-méthylpipérazin-1-ylméthyl)-1,2,3,11-tétrahydro-5,11-diazabenzo[a]trindène-4,6-dione, ou d'une forme de sel pharmaceutiquement acceptable de celle-ci.

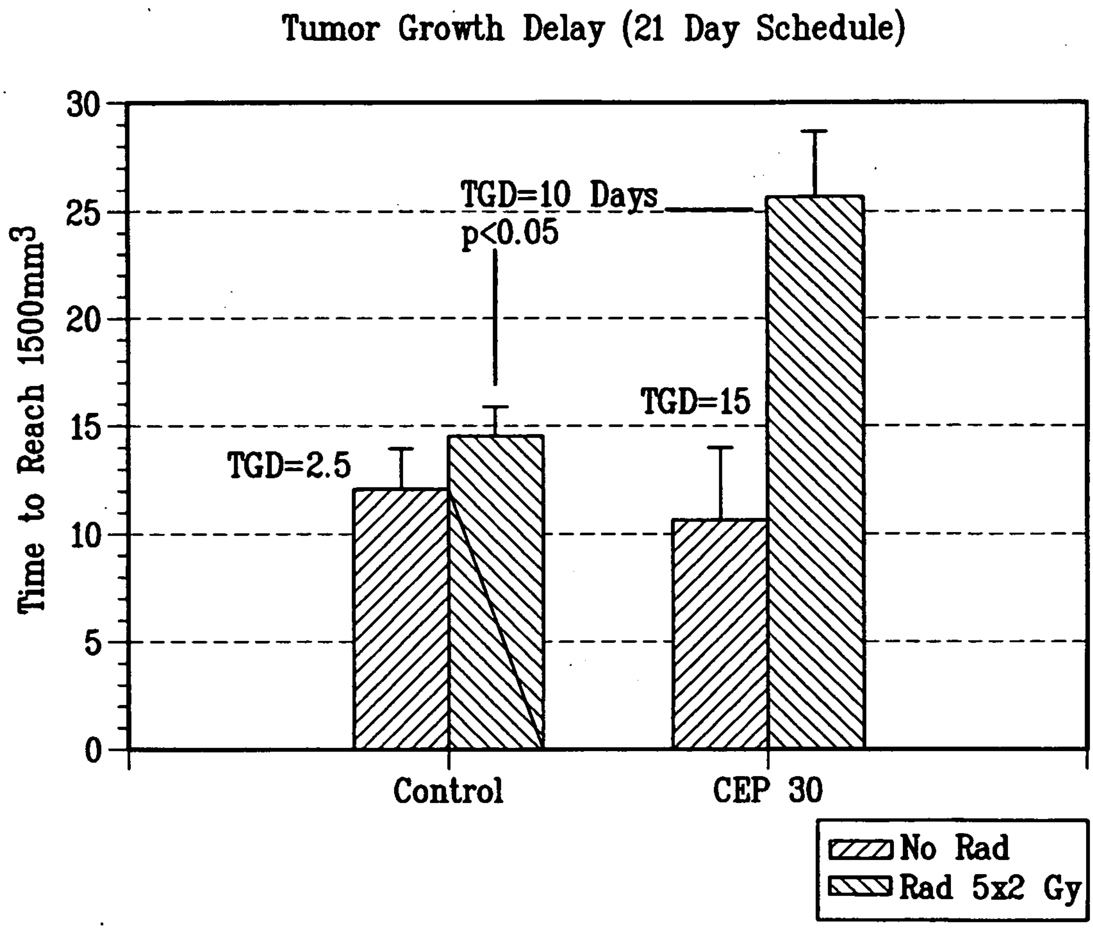
Tumor Growth Delay (21 Day Schedule)
Time to Reach 1500mm3
30
25
20
15
10
5
0
TGD=10 Days
p<0.05
TGD=15
TGD=2.5
Control
CEP 30
No Rad
Rad 5x2 Gy

*FIG. 1*

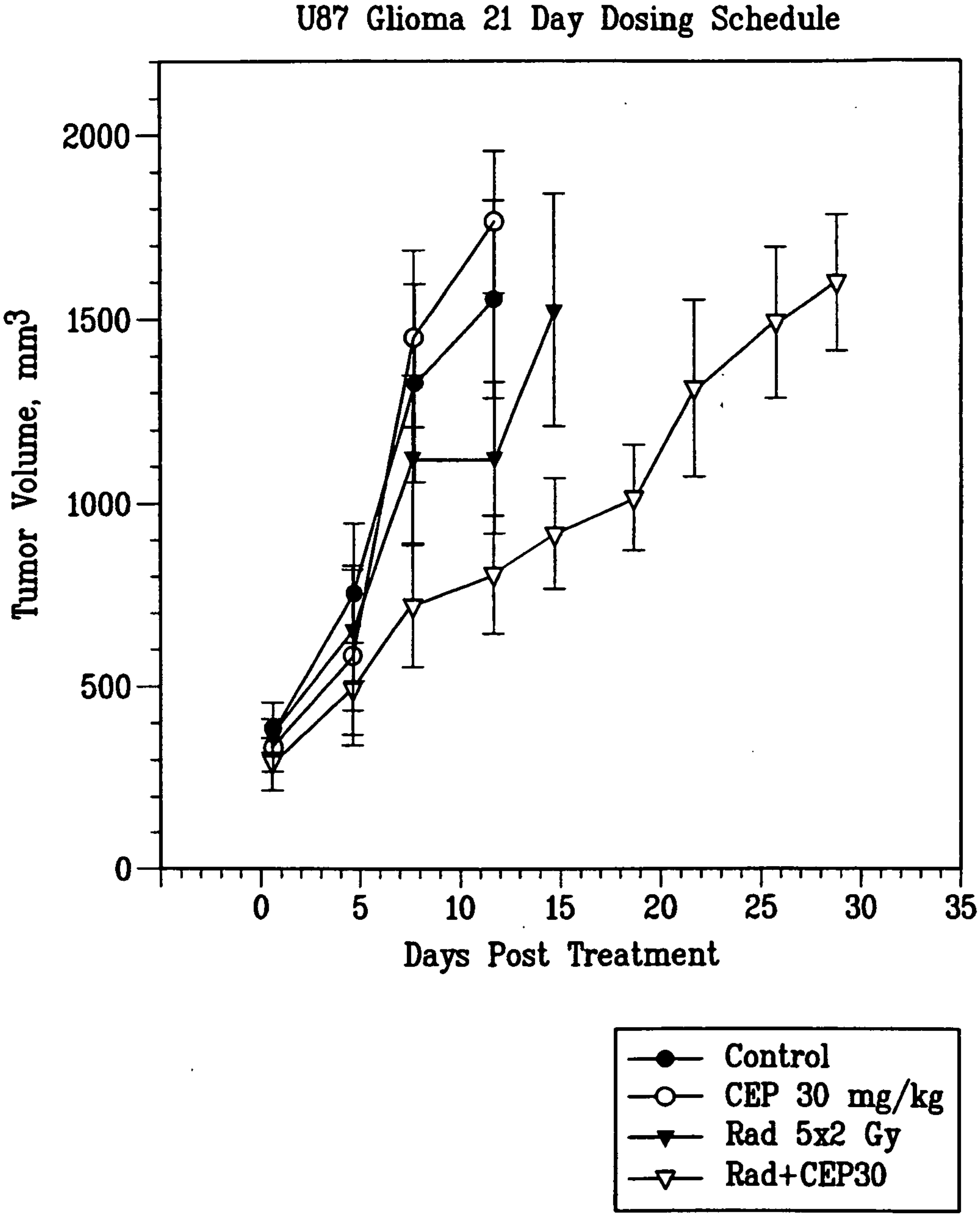
U87 Glioma 21 Day Dosing Schedule
Tumor Volume, mm3
0
500
1000
1500
2000
0
5
10
15
20
25
30
35
Days Post Treatment
Control
CEP 30 mg/kg
Rad 5x2 Gy
Rad+CEP30

FIG. 2

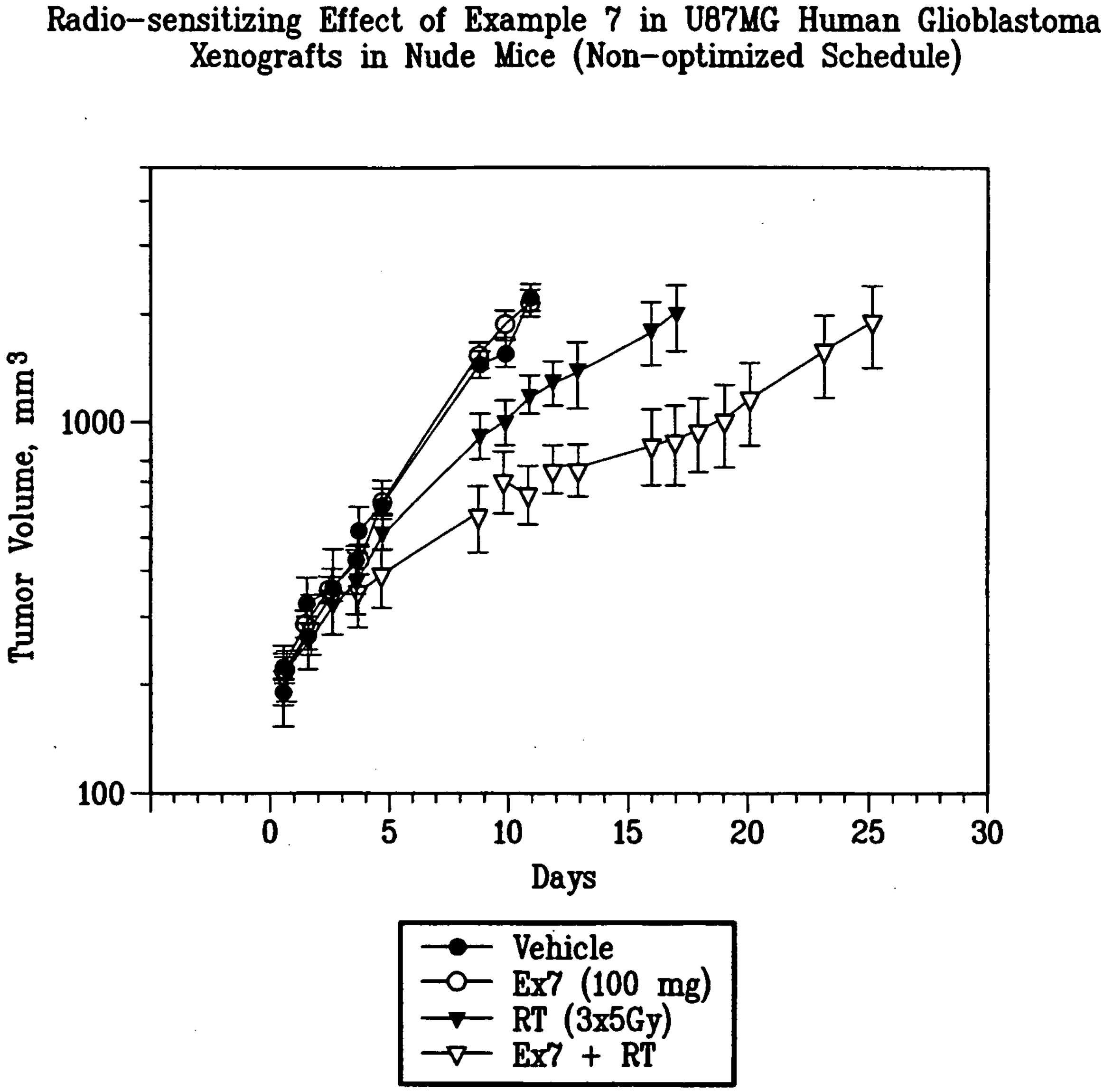

Radio-sensitizing Effect of Example 7 in U87MG Human Glioblastoma Xenografts in Nude Mice (Non-optimized Schedule)
Tumor Volume, mm3
1000
100
0
5
10
15
20
25
30
Days
Vehicle
Ex7 (100 mg)
RT (3x5Gy)
Ex7 + RT


*FIG. 3*

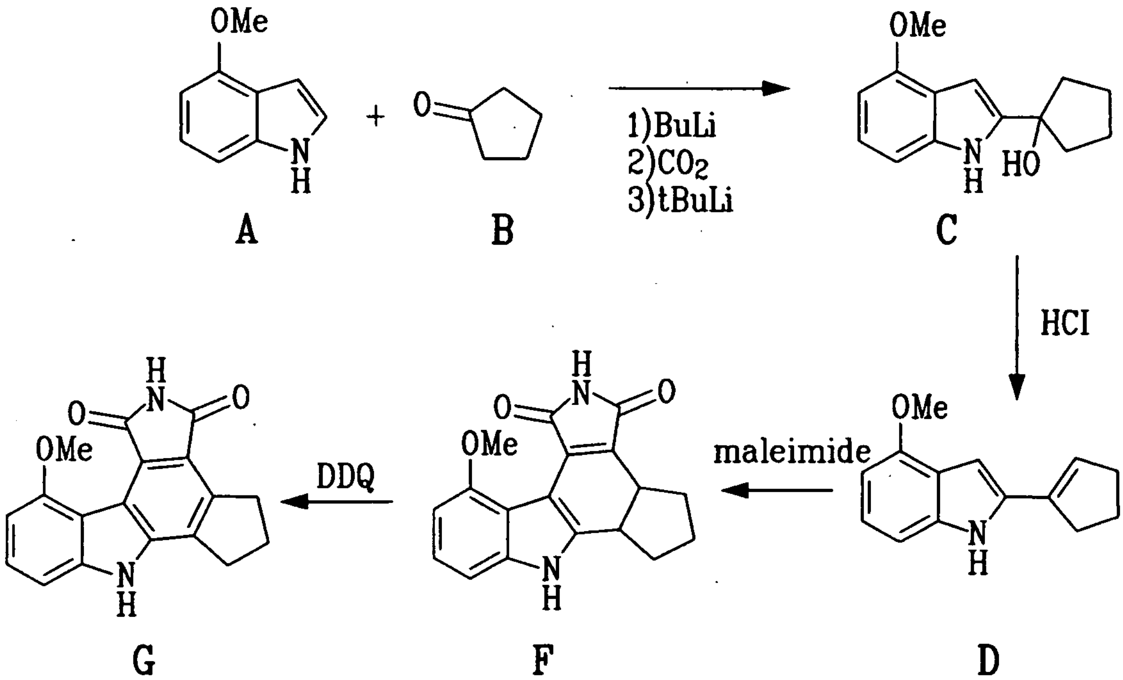


FIG. 4

Radio-sensitizing Effect of Example 7 Administered Orally in U87MG Human Glioblastoma Xenografts in Nude Mice.

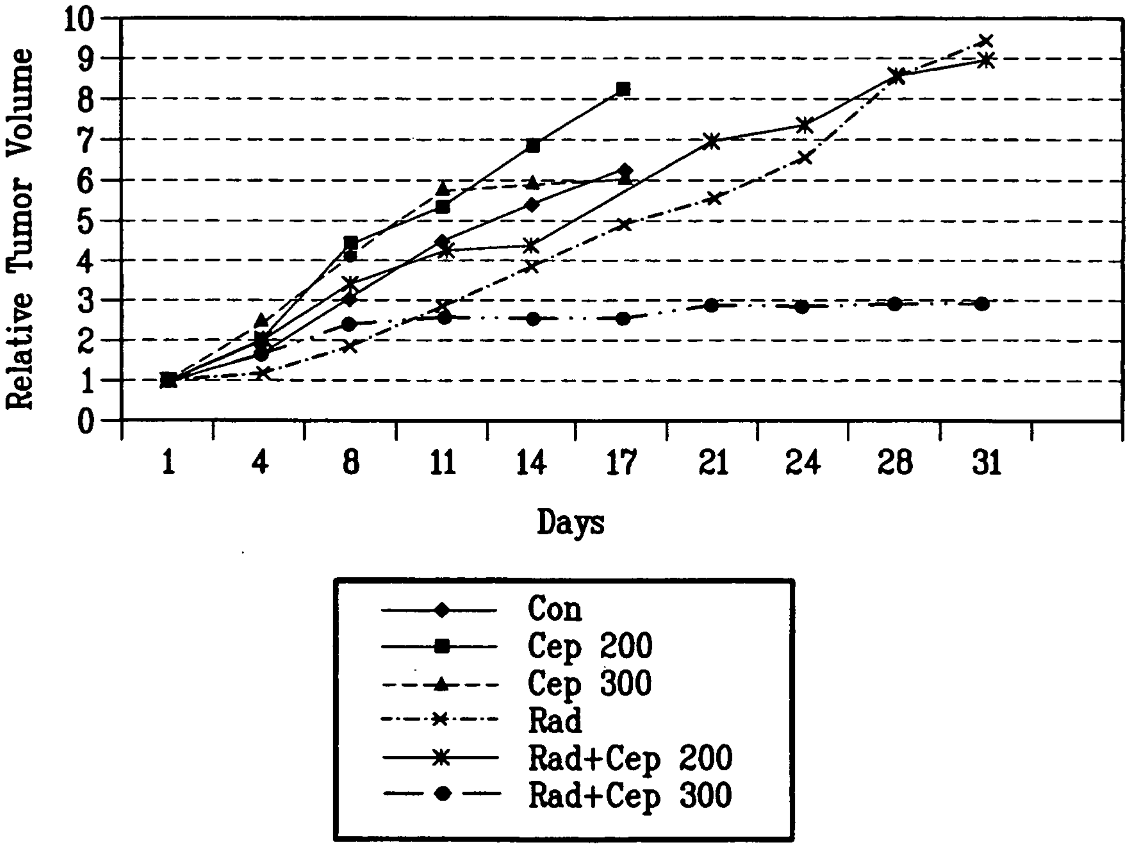


FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001085686 A


### Non-patent literature cited in the description


- Prodrugs, Sloane. Marcel Dekker, 1992
- **Purnell ; Whish.** *Biochem. J.,* 1980, vol. 185, 775
- **Curtin, NJ.** PARP inhibitors for cancer therapy. *Expert Rev Molec Med,* 2005, vol. 4, 1-20
- **Griffin, R et al.** Resistance-modifying agents. 5. Synthesis and biological properties of quinazolinone inhibitors of the DNA repair enzyme poly(ADP-ribose)polymerase (PARP). *J. Med. Chem.,* 1998, vol. 42, 5247-5256
- **Bowman, KJ et al.** Potentiation of anti-cancer agent cytotoxicity by the potent poly(ADP-ribose)polymerase inhibitors NU 1025 and NU 1064. *Br. J, Cancer,* 1998, vol. 78, 1269-1277
- **Bowman, KJ et al.** Differential effects of the poly(ADP-ribose)polymerase inhibitor NU 1025 on topoisomerase I and II inhibitor cytotoxicity in L1210 cells in vitro. *Br. J. Cancer,* 2001, vol. 84, 106-112
- **Chen ; Pan.** Potentiation of antitumor activity of cisplatin in mice by 3-aminobenzamide and nictotinamide. *Cancer Chemoth. Pharmacol.,* 1988, vol. 22, 303-307
- **Delaney, CA et al.** Potentiation of temozolomide and topotecan growth inhibition and cytotoxicity by novel poly(adenosine diphosphoribose)polymerase inhibitors in a panel of human tumor lines. *Clin. Cancer Res.,* 2000, vol. 6, 2860-2867
- **Griffin R et al.** The role of inhibitors of poly(ADP-ribose)polymerase as resistance-modifying agents in cancer therapy. *Biochimie,* 1995, vol. 77, 408-422
- **Liu, L et al.** Pharmacologic disruption of base excision repair sensitizes mismatch repair-deficient and-proficient colon cancer cells to methylating agents. *Clin. Cancer Res.,* 1999, vol. 5, 2908-2917
- **Tentori, L. et al.** Combined treatment with temozolomide and poly(ADP-ribose) polymerase inhibitor enhances survival of mice bearing hematologic malignancy at the central nervous system site. *Blood,* 2002, vol. 15, 2241-2244
- **Baldwin J.** FDA evaluating oxaliplatin for advance colorectal cancer treatment. *J. Natl. Cancer Inst.,* 2002, vol. 94, 1191-1193
- **Jagtap P ; Szabo C.** Poly (ADP-Ribose) polymerase and the therapeutic effects of its inhibitors. *Nature Rev Drug Disc,* 2005, vol. 4, 421-440
- **Ame JC et al.** The PARP superfamily. *Bioessays,* 2004, vol. 26, 882-893
- **Smith, S.** The World According to PARP. *Trends Biochm.Sci.,* 2001, vol. 26, 174-179
- **Virag, L. ; Szabo, C.** The therapeutic potential of poly(ADP-ribose) polymerase inhibitors. *Pharmacol. Rev.,* 2002, vol. 54, 375-429
- **de Murcia, JM et al.** Requirement of poly(ADP-ribose)polymerase in recovery from DNA damage in mice and cells. *Proc Natl Acad Sci USA,* 1997, vol. 94, 7303-7307
- **Masuntani, M et al.** The response of PARP knockout mice against DNA damaging agents. *Mutat. Res.,* 2000, vol. 462, 159-166
- **Kato, T et al.** Enhancement of bleomycin activity by 3-aminobenzamide, a poly(ADP-ribose) synthesis inhibitor, in vitro and in vivo. *Anticancer Res.,* 1988, vol. 8, 239-244
- **Smith, L. et al.** The novel poly(ADP-Ribose) polymerase inhibitor, AG14361, sensitizes cells to topoisomerase I poisons by increasing the persistence of DNA strand breaks. *Cl Cancer Res.,* 2005, vol. 11, 8449-8457